# FASCICULE DE BREVET EUROPEEN

(11) **EP 3 191 516 B1**
(45) Date de publication et mention de la délivrance du brevet: **26.02.2020**
(21) Numéro de dépôt: 15771201.9
(22) Date de dépôt: 10.09.2015
(51) Int. Cl.: C07K 16/26, A61K 39/395, A61K 38/24, A61P 5/06, A61P 5/24, A61P 15/08

(54) **LIGAND POTENTIALISANT LA BIOACTIVITE DE LA FSH**
LIGAND ZUR STÄRKUNG DER FSH-BIOAKTIVITÄT
LIGAND POTENTIALIZING FSH BIOACTIVITY

(30) Priorité: 10.09.2014 FR 1458463
(43) Date de publication de la demande: 19.07.2017
(73) Titulaire: ReproPharm Vet, 37380 Nouzilly (FR); Institut National de la Recherche Agronomique (INRA), 75007 Paris (FR)
(72) Inventeur: MAUREL, Marie-Christine, 37000 Tours (FR); REITER, Eric, 37380 Nouzilly (FR); JEGOT, Gwenhael, 29840 Landunvez (FR); DURAND, Guillaume, 33400 Talence (FR); KARA, Elodie, 37250 Veigne (FR); CASTERET, Sophie, 37190 Valleres (FR); POUPON, Anne, 41100 Mazange (FR)
(74) Mandataire: Novagraaf Technologies
(86) Numéro de dépôt international: PCT/FR2015/052415
(87) Numéro de publication internationale: WO 2016/038310

(56) Documents cités:
- WO-A1-97/12038
- WO-A1-2012/066519
- US-A1- 2002 102 613
- GLENCROSS R G ET AL: "Monoclonal antibody enhancement of FSH-induced uterine growth in snell dwarf mice", JOURNAL OF ENDOCRINOLOGY, SOCIETY FOR ENDOCRINOLOGY, GB, vol. 136, no. 3, 1 mars 1993 (1993-03-01), pages R5-R7, XP009145848, ISSN: 0022-0795, DOI: 10.1677/JOE.0.136R005
- ALFREDO ULLOA-AGUIRRE ET AL: "Novel pathways in gonadotropin receptor signaling and biased agonism", REVIEWS IN ENDOCRINE AND METABOLIC DISORDERS, KLUWER ACADEMIC PUBLISHERS, BO, vol. 12, no. 4, 28 avril 2011 (2011-04-28) , pages 259-274, XP019972343, ISSN: 1573-2606, DOI: 10.1007/S11154-011-9176-2
- MILLER K F ET AL: "Immunoaffinity chromatography of bovine FSH using monoclonal antibodies", JOURNAL OF ENDOCRINOLOGY, SOCIETY FOR ENDOCRINOLOGY, GB, vol. 115, no. 2, 1 novembre 1987 (1987-11-01), pages 283-288, XP009187286, ISSN: 0022-0795

## Description

### Domaine technique

La présente invention se rapporte à des anticorps dirigés contre l'hormone folliculo-stimulante (FSH) capables de potentialiser la bioactivité de la FSH mais pas celle de l'hormone lutéinisante (LH).

La présente invention trouve ses applications principalement en médecine vétérinaire, pour induire l'ovulation chez un mammifère femelle.

Dans la description ci-dessous, les références entre crochets (**[ ]**) renvoient à la liste des références présentée à la fin du texte.

### Etat de la technique

Les gonadotrophines (ou gonadotropines) sont des hormones glycoprotéiques complexes jouant un rôle central dans la régulation de la reproduction chez les vertébrés en agissant sur les fonctions des gonades (ovaires et testicules). Deux de ces hormones sont sécrétées chez tous les vertébrés : l'hormone lutéinisante (LH) et l'hormone folliculo-stimulante (FSH). Chez deux groupes de mammifères, équidés et primates, il existe en outre une gonadotrophine chorionique (CG) sécrétée par le placenta : la choriogonadotropine humaine (hCG) et la choriogonadotropine équine (eCG) qui agissent toutes deux via des récepteurs LH.

L'hormone lutéinisante (LH) est produite par les cellules gonadotropes du lobe antérieur de l'hypophyse sous stimulation de la GnRH, elle-même produite par l'hypothalamus. La LH stimule la production de testostérone chez les mâles, tandis qu'elle intervient dans les modifications du cycle ovarien chez les femelles où elle est responsable de la croissance folliculaire terminale et de l'ovulation puis de la transformation du follicule ovulatoire rompu en corps jaune. Pendant la phase lutéale du cycle menstruel, la LH stimule la sécrétion de progestérone par le corps jaune, indispensable au développement précoce et à l'implantation de l'embryon. La LH est constituée d'une sous-unité α commune à toutes les hormones glycoprotéiques d'une même espèce (comme la FSH, la CG et l'hormone thyréostimulante, la TSH) et d'une sous-unité β responsable de la spécificité d'activité de l'hormone ; activité qui n'existe que si les deux sous-unités sont associées de manière non covalente sous-forme d'un dimère.

L'hormone folliculo-stimulante (ou FSH) est produite par l'anté-hypophyse sous stimulation de la GnRH produite par l'hypothalamus. Chez les mâles, elle stimule les cellules de Sertoli indispensables à la spermatogénèse. Chez les femelles, elle est responsable du recrutement des follicules primordiaux, immatures, de leur croissance et de leur différentiation en follicules pré-ovulatoires en stimulant les récepteurs FSH des cellules de la granulosa. La FSH est constituée de deux sous-unités α et β, et a une structure semblable à celle de la LH. Seul le dimère est capable de stimuler les récepteurs FSH.

Chez les femelles, les taux de LH et de FSH sont cycliques : très faibles en période de repos sexuel ou en dehors de la période ovulatoire, avec un pic de sécrétion en période préovulatoire.

Les gonadotrophines sont utilisées en médecine vétérinaire et humaine, pour induire l'ovulation chez les mammifères femelles. Bien qu'efficaces, ces traitements présentent un risque sanitaire du fait de l'utilisation d'hormones extraites à partir de fluides biologiques (sang, urine) ou de tissus (hypophyses), particulièrement dans le domaine vétérinaire. C'est le cas de la chorionic gonadotropine équine (eCG) extraite à partir de sang de juments gravides, et de la LH et FSH porcines extraites à partir d'hypophyses de porc. Dans le domaine vétérinaire, on utilise également une hCG extraite à partir d'urine de femmes enceintes, le Chorulon® (Laboratoire MSD).

Dans le domaine de la clinique humaine, et particulièrement de la Procréation Médicalement Assistée (ou PMA), on utilise des hormones extraites à partir d'urine de femmes ménopausées telles que Fostimon® (Laboratoire Genévrier) qui est une FSH purifiée et Menopur® (Laboratoire Ferring Pharmaceuticals) qui est une hMG (human menoposal gonadotropin), mélange de FSH et de LH et la Gonadotropine Chorionique Endo5000® qui est une hCG purifiée (Laboratoire Schering-Plough). On utilise également des FSH humaines recombinantes, telles que Gonal-F® (Laboratoire Merck Serono) et Puregon® (Laboratoire Merck Shering-Plough); des hCG et LH recombinantes telle que Ovidrel® et Luveris® (Laboratoire Merck Serono).

En outre l'usage répété de ces hormones induit le plus souvent une réaction immunitaire qui vient neutraliser l'effet des hormones conduisant ainsi à une baisse d'efficacité thérapeutique. Cependant, il a également été mis en évidence dans quelques cas que la réaction immunitaire pouvait produire des anticorps capables de potentialiser l'activité de l'hormone lorsqu'elle était co-administrée (Brevet EP 1518863) [1]. Depuis, il a également été mis en évidence trois anticorps monoclonaux anti-LH capables de potentialiser son action ainsi que celle de la FSH pour deux d'entre eux (Demande Internationale WO 2012/066519) [2].

### Description de l'invention

Les Inventeurs ont maintenant obtenu un anticorps monoclonal produit contre la FSH ovine (oFSH). Cet anticorps est capable de potentialiser son action ainsi que celle de la FSH humaine recombinante Gonal-F®, de la choriogonadotropine équine (eCG) et humaine (hCG) Chorulon® et Endo 5000®.

Cet anticorps monoclonal est dénommé A8.

L'hybridome qui a produit l'anticorps A8 a été déposé conformément au Traité de Budapest, le 6/12/2013 auprès de la CNCM (Collection Nationale de Culture de Microorganismes, Institut Pasteur, 25 rue du Docteur Roux, 75724 Paris Cedex 15, France), sous le numéro CNCM I-4828.

Les séquences nucléotidiques des régions variables des chaines lourdes et légères de l'anticorps A8 ont été déterminées, les séquences peptidiques correspondantes déduites, et sont présentées dans le tableau 1 ci-dessous.

**Tableau 1**

| Anticorps monoclonal A8 | |
|---|---|
| Chaîne lourde (VH) | |
| Séquence nucléotidique (SEQ ID NO : 1) | |
| Séquence peptidique (SEQ ID NO : 2) | |

| Chaîne légère (VL) | |
|---|---|
| Séquence nucléotidique (SEQ ID NO : 3) | |
| Séquence peptidique (SEQ ID NO : 4) | |

Les séquences codant pour les CDRs (régions déterminant la complémentarité) ont été déterminées à partir des séquences des régions variables des chaînes lourdes (VH-CDR) et légères (VL-CDR) des anticorps A8 ci-dessus. Les séquences peptidiques correspondantes ont été déduites, et sont présentées respectivement dans le tableau 2 ci-dessous.

**Tableau 2**

| Anticorps monoclonal A8 | |
|---|---|
| VH-CDR1 (SEQ ID NO : 5) | GFTFSDFG |
| VH-CDR2 (SEQ ID NO : 6) | ISSGSSTR |
| VH-CDR3 (SEQ ID NO : 7) | ARSYDGYHVPSFAY |
| VL-CDR1 (SEQ ID NO : 8) | QNIVHSNGNTY |
| VL-CDR2 | RVS |
| VL-CDR3 (SEQ ID NO : 9) | FQGSHVPLT |

La présente invention a pour objet un ligand de l'hormone folliculo-stimulante (FSH) potentialisant la bioactivité de la FSH et de la gonadotropine chorionique (CG) mais pas celle de l'hormone lutéinisante (LH), caractérisé en ce que :
le domaine variable de la chaine lourde contient les CDRs suivants :
- VH-CDR1, défini par la séquence GFTFSDFG (SEQ ID NO : 5) ;
- VH-CDR2, défini par la séquence ISSGSSTR (SEQ ID NO : 6) ;
- VH-CDR3, défini par la séquence ARSYDGYHVPSFAY (SEQ ID NO : 7) ; et le domaine variable de la chaine légère contient les CDRs suivants :
- VL-CDR1, défini par la séquence QNIVHSNGNTY (SEQ ID NO : 8) ;
- VL-CDR2, défini par la séquence RVS ;
- VL-CDR3, défini par la séquence FQGSHVPLT (SEQ ID NO : 9).

On entend par « CDR » au sens de la présente invention, les trois régions hypervariables des régions variables des chaînes lourdes et légères d'un anticorps qui constituent les éléments du paratope et permettent de déterminer la complémentarité de l'anticorps avec l'épitope de l'antigène. Ces trois régions hypervariables sont encadrées par quatre régions constantes qui constituent la « charpente » (FR ou framework régions) et donne une configuration stable au domaine variable.

Un ligand selon la présente invention est par exemple :
- l'anticorps monoclonal A8 produit par l'hybridome CNCM I-4828
- un fragment Fab, Fab', F(ab')2, Fv, dsFv ou scFv, un nanobody de l'anticorps ci-dessus. De préférence, il s'agit d'un fragment scFv ;
- une forme bi-, tri- ou tétravalente (diabodies, triabodies, tétrabodies) de deux, trois ou quatre fragments de scFv, respectivement ;
- un anticorps recombinant comprenant le paratope de l'anticorps ci-dessus et dont les régions constantes ont été modifiées de sorte à minimiser l'immunogénicité vis-à-vis de l'animal ou de l'homme auquel il est destiné. Par exemple, il s'agit d'un anticorps chimérique (humanisé, ovinisé, caprinisé, bovinisé, porcinisé etc...) ou entièrement humanisé, ovinisé, caprinisé, bovinisé, porcinisé.

A titre d'exemple non limitatif, la séquence nucléotidique du scFv dérivé de l'anticorps A8 a été déterminée, la séquence peptidique correspondante déduite, et sont présentées dans le tableau 3 ci-dessous.

**Tableau 3**

| scFv A8 | |
|---|---|
| Séquence nucléotidique (SEQ ID NO : 10) | |
| | |
| Séquence peptidique (SEQ ID NO 11) | |

La présente invention a également pour objet une séquence nucléotidique codant pour un ligand selon l'invention.

La présente invention a également pour objet un vecteur recombinant, en particulier un vecteur d'expression, comprenant une séquence nucléotidique selon l'invention.

La présente invention a également pour objet une cellule hôte comprenant une séquence nucléotidique selon l'invention ou un vecteur recombinant selon l'invention. Par exemple, il s'agit de l'hybridome CNCM I-4828 ou d'une cellule transformée par une séquence nucléotidique ou un vecteur recombinant selon l'invention.

La présente invention a également pour objet un procédé de production d'un ligand selon l'invention, caractérisé en ce qu'il comprend la mise en culture dans un milieu approprié de cellules hôte selon l'invention, et la récupération dudit ligand à partir de ladite culture.

Les Inventeurs ont mis en évidence que l'anticorps A8 potentialise la FSH ovine, la FSH humaine recombinante Gonal-F®, la eCG et les hCG Chorulon® et Endo 5000®. En outre, les Inventeurs ont mis en évidence que le scFv dérivé de l'anticorps A8 possède les mêmes propriétés de liaison et de potentialisation que l'anticorps dont il dérive.

La présente invention a également pour objet un ligand selon l'invention pour une utilisation comme médicament, en particulier pour potentialiser la bioactivité de la FSH, et de la gonadotropine chorionique (CG) pour induire l'ovulation chez un mammifère femelle et pour réduire les problèmes d'infertilité ou d'hypofertilité hormono dépendants chez un mammifère mâle ou femelle.

La présente invention a également pour objet un complexe formé d'un ligand et d'une gonadotrophine, ou d'un peptide actif de celle-ci, capable de se lier audit ligand et dont l'activité est potentialisée par ledit ligand. Par exemple, il s'agit du complexe d'un ligand avec l'hormone gonadotropine chorionique (CG) ou avec la FSH, extraites de tissus ou fluides biologiques ou recombinantes, ou un peptide actif de celles-ci capable de se lier audit ligand et dont l'activité est potentialisée par ledit ligand.

La présente invention a également pour objet un ligand ou complexe selon l'invention pour une utilisation comme médicament, en particulier pour induire une (mono)ovulation voire une polyovulation chez un mammifère femelle ou pour réduire les problèmes d'infertilité ou d'hypofertilité hormono dépendants chez un mammifère mâle ou femelle. Ledit médicament permet également d'augmenter le taux de progestérone endogène circulant sécrété par un ou plusieurs corps jaunes chez un mammifère femelle, favorisant ainsi le développement embryonnaire précoce et diminuant le risque d'avortement.

La présente invention a également pour objet un procédé de production carnée, où ledit procédé comprend l'administration de ligand et/ou de complexe de l'invention à un mammifère femelle animal non humain.

La présente invention a également pour objet un ligand et/ou complexe de l'invention pour une utilisation dans le traitement de l'infertilité ou de l'hypofertilité hormono dépendante chez un mammifère. Dans le cas d'un mammifère femelle souffrant d'infertilité ou d'hypofertilité, l'administration du ligand ou complexe de l'invention va permettre de stimuler une procréation naturelle, médicalement assistée ou artificielle. Il est à noter que l'administration du ligand ou complexe de l'invention à un mammifère femelle sain va également permettre de déclencher l'ovulation dans le cadre d'une procréation naturelle ou artificielle.

On entend par « infertilité/hypofertilité hormono dépendante » au sens de la présente invention, une infertilité/hypofertilité due à une insuffisance hormonale par exemple de faibles concentrations circulantes de FSH ou une absence de cette hormone résultant par exemple d'une cause externe (par exemple les pesticides) ou interne (par exemple, insuffisance hypophysaire ou hypothalamique ou d'un problème de réceptivité des gonades à la FSH due à une anomalie des récepteurs ou des gonadotropines FSH, CG par exemple une mutation ou un polymorphisme des récepteurs).

Les ligands et complexes de l'invention peuvent être utilisés chez l'homme ou l'animal, en particulier les ovins, bovins, caprins, équins, porcins, murins, canins, camelins etc...

Les ligands, les hormones ou les complexes selon l'invention peuvent être administrés soit séparément, soit séquentiellement, soit conjointement, par injection, par exemple intramusculaire, intraveineuse, intrapéritonéale, sous-cutanée, transcutanée, intradermique, intraorbitaire, intraoculaire, ophtalmique, ou par voie transoculaire, sans altérer leur effet potentialisant.

La présente invention a également pour objet une composition pharmaceutique comprenant un ligand ou complexe de l'invention et un véhicule pharmaceutiquement acceptable. Ladite composition pharmaceutique peut comprendre en outre de la FSH et/ou de l'hormone gonadotropine chorionique (CG).

D'autres avantages pourront encore apparaître à l'homme du métier à la lecture des exemples ci-dessous, illustrés par les figures annexées, donnés à titre illustratif.

### Brève description des figures

- La figure 1 représente les sensorgrammes d'interaction de l'anticorps A8 avec la FSH ovine (A) et la FSH humaine gonal F (B). Les 6 sensorgrammes représentés dans les figures A et B sont ceux obtenus pour les différentes concentrations de oFSH et hFSH. La répétition de chaque injection est représentée pour chaque concentration.
- La figure 2 représente l'effet potentialisant *in vitro* de l'anticorps monoclonal A8 sur la bioactivité de la FSH ovine (oFSH) sur des cellules de granulosa bovines (A) et sur une lignée cellulaire HEK 293 transfectée de façon stable avec le récepteur FSH humain (B). Elle illustre également l'effet potentialisant de l'anticorps A8 sur la bioactivité de la FSH humaine (hFSH) Gonal F® sur la lignée cellulaire HEK 293 transfectée de façon stable avec le récepteur FSH humain (C).
- La figure 3 représente représente l'effet potentialisant *in vitro* de l'anticorps monoclonal A8 (A) et du scFv A8 (B) sur la bioactivité de la FSH ovine en utilisant une lignée cellulaire HEK 293 transfectée de façon stable avec le récepteur FSH humain et le vecteur Glosensor®, et l'effet de l'anticorps A8 sur la FSH humaine (hFSH) Gonal F® (C).
- La figure 4 représente l'effet potentialisant *in vivo* de l'anticorps monoclonal A8 sur la bioactivité de la FSH ovine (oFSH) (A) et sur la bioactivité de la FSH humaine (hFSH) Gonal F® (B) chez le rat femelle.
- La figure 5 représente l'effet potentialisant *in vivo* de l'anticorps monoclonal A8 et du scFv A8 sur la bioactivité de la FSH humaine Gonal F® (hFSH) selon une administration par voie intra-péritonéale chez le rat femelle.
- La figure 6 représente l'effet potentialisant *in vivo* de l'anticorps monoclonal A8 sur la bioactivité des choriogonadotropines humaines (hCG) Chorulon® et Endo 5000® chez le rat mâle.
- La figure 7 représente l'effet potentialisant *in vivo* de l'anticorps monoclonal A8 sur la bioactivité de la choriogonadotropine équine (eCG) Chronogest® chez le rat femelle.
- La figure 8 représente l'effet potentialisant *in vivo* de l'anticorps monoclonal A8 injecté seul sur la bioactivité des gonadotropines endogènes chez la brebis en début de saison sexuelle.
- La figure 9 représente l'effet potentialisant *in vivo* du complexe FSH porcine (pFSH)/A8 et celui obtenu avec l'anticorps monoclonal A8 injecté seul chez la brebis en période de saison sexuelle.
- La figure 10 représente l'épitope conformationnel du ligand A8 sur les hormones hFSH, hCG, hLH, oLH, pLH, oFSH, pFSH et sur le récepteur FSH humain.

### EXEMPLES

### EXEMPLE 1 : OBTENTION DES LIGANDS DE L'INVENTION, ET LEUR CARACTERISATION

### Stratégie d'immunisation des souris

Cinq souris Balb/C ont été immunisées contre la FSH ovine (oFSH). Les injections ont été réalisées par voie intra-péritonéale et par voie intra-dermique selon la stratégie d'immunisation suivante :
- J0 : une première injection par voie intra-dermique (J0) a été réalisée avec 50µg de FSH ovine (purifiée avec adjuvant de Freund complet ;
- J23 : injection par voie intra-péritonéale par voie intra-péritonéale de 30µg de oFSH purifiée avec adjuvant de Freund Incomplet ;
- J44 : injection par voie intra-dermique de 30µg de FSH ovine purifiée avec adjuvant de Freund incomplet ;
- J65 : injection par voie intra-péritonéale de 30µg de sous-unité béta (β) de oFSH avec adjuvant de Freund incomplet ;
- J82 : injection par voie intra-péritonéale de 30µg de sous-unité β de FSH ovine sans adjuvant ;
- J86: fusion.

### Isotypage

L'isotypage de l'anticorps A8 a été réalisé avec le kit d'isotypage FastElysa commercialisé par RD Biotech (référence RDB 3255) en suivant les recommandations du fabriquant.

L'anticorps A8 est une immunoglobine de classe IgG1 et d'isotype Kappa. Les valeurs des densités optiques (DO) obtenues ont été 0,288 et 0,185 respectivement.

### Séquençage

Les séquences nucléotidiques de la partie variable de la chaîne lourde (VH) et légère (VL) de l'anticorps A8 sécrété par l'hybridome CNCM I-4828 ont été déterminées à partir de leur ARN messager (ARNm) selon le protocole ci-après. Les ARNs ont été extraits des cellules à l'aide du kit RNeasy® Mini Kit (Ref 74104, Qiagen, Pays-Bas) en suivant les recommandations du fabriquant. Les concentrations en ARN purifiés ont été estimées par mesure de l'absorbance (A) à 260 nm et leur qualité par le rapport A260nm/280nm et visuellement après migration électrophorétique sur gel d'agarose.

Les ADN complémentaires des ARNm ont alors été synthétisés à l'aide d'un oligo-dT₁₈ comme amorce par réaction de rétrotranscription avec l'enzyme M-MLV (Ref M1701, Promega, USA) en suivant les recommandations du fabriquant.

La synthèse du second brin d'ADN a été réalisée par une réaction de polymérisation en chaîne (PCR) selon le protocole suivant : à 4 µl de la réaction de rétrotranscription sont ajoutés dans un volume final de 50 µl ; le tampon de réaction (1X final), 200 µM de chaque dNTPs, 300 nM d'amorces sens et antisens, 1,25 U de GoTaq polymérase (Ref M3175, Promega, USA).

Pour l'amplification de la partie variable de la chaîne légère, 2 couples d'amorces différents ont été utilisés (MKRev1+ MKC5For et MKRev 7 + MKC5For) et 3 couples différents pour celle de la chaîne lourde (VHRev1 + VHFor1, VHRev1 + VHFor2 et VHRev2 + VHFor3).

**Tableau 4: Séquences nucléotidiques des amorces utilisées pour séquencer les chaînes lourdes (VH) et légères(VL) de l'anticorps A8**

| Anticorps A8 | | |
|---|---|---|
| Chaîne lourde (VH) | | |
| **Nom** | **Séquence 5'-3'** | **SEQ ID NO** |
| **VHRev1** | | SEQ ID NO : 12 |
| **VHRev2** | | SEQ ID NO : 13 |
| **VHFor1** | CAGGGGCCAGTGGATAGAC | SEQ ID NO :14 |
| **VHFor2** | CGGGATCCTCTAGACAGTGGATARACMGATGG | SEQ ID NO : 15 |
| **VHFor3** | | SEQ ID NO : 16 |

| Chaîne légère (VL) | | |
|---|---|---|
| **MKRev1** | GATGTTTTGATGACCCAAACT | SEQ ID NO : 17 |
| **MKRev7** | GATATCCAGATGACACAGACT | SEQ ID NO : 18 |
| **MKC5For** | GGATACAGTTGGTGCAGCATC | SEQ ID NO : 19 |

Le programme de PCR qui a été utilisé est composé d'une dénaturation initiale de 2 min à 95°C suivie de 30 cycles de dénaturation 30 sec à 95°C, hybridation 30 sec à 47°C et amplification 1 min à 72°C et enfin d'une amplification finale de 5 min à 72°C. Les produits de PCR obtenus ont été dessalés avec le kit QIAquick®Gel extraction kit (Ref 28704, Qiagen GmbH, Allemagne) puis ligaturés avec le plasmide pGEMT easy vector (ref A1360, Promega, USA) pour être transformés en bactéries. L'ADN plasmidique extrait de différents clones bactériens a été envoyé pour analyse par séquençage (Macrogen Europe, Pays-Bas).

La séquence nucléotidique 5' terminale de la chaîne légère de l'anticorps a été déterminée grâce à la conception d'amorces spécifiques ancrées dans la séquence leader de l'ADNc (amorce Fw). Trois amorces ont été conçues suite à l'identification d'homologie par alignement entre la séquence VL obtenue précédemment et la base de données ENSEMBL (release 64 ; http://uswest.ensembl.org/index.html, dans le domaine public). L'amorces antisens (Rev) a été conçue dans la séquence VL précédemment déterminée. Le protocole utilisé pour obtenir la partie 5' est le même que celui décrit au paragraphe précédent.

**Tableau 5 Séquences nucléotidiques des amorces utilisées pour séquencer la partie 5'de la chaîne légère (VL) de l'anticorps A8**

| Anticorps A8 | | |
|---|---|---|
| **Nom** | **Séquence 5'-3**' | **SEQ ID N0** |
| Chaîne légère (VL) | | |
| **A8_VL4** | ATGAAGTTGCCTGTTAGGCTGTTGGTGCTG | SEQ ID NO :20 |
| **A8-VL5** | GCTGATGTTCTGGATTCCTGCTTCCAGCAGT | SEQ ID NO :21 |
| **A8-VL6** | ATGATGAGTCCTGCCCAGTTCCTGTTTCTG | SEQ ID NO :22 |
| **A8-VLrev** | TGTGAGCGGAACATGTGAACCTTGAAA | SEQ ID NO :23 |

Les séquences nucléotidiques consensus ont été déduites de l'alignement des séquences en utilisant le logiciel MultAlin (Corpet, Nucl. Acids Res., 16(22) :10881-10890, 1988) [3]. La transcription en séquences polypeptidiques et l'annotation des CDRs ont été réalisées à l'aide de logiciel IMGT/V-QUEST. Les résultats sont présentés dans les tableaux 6 et 7 .

**Tableau 6 : Séquences nucléotidiques et peptidiques des parties variables lourdes (VH et légères(VL) de l'anticorps A8**

| Anticorps monoclonal A8 | |
|---|---|
| Chaîne lourde (VH) | |
| Séquence nucléotidique (SEQ ID NO : 1) | |
| Séquence peptidique (SEQ ID NO : 2) | |

| Chaîne légère (VL) | |
|---|---|
| Séquence nucléotidique (SEQ ID NO : 3) | |
| Séquence peptidique (SEQ ID NO : 4) | |

**Tableau 7: CDR des parties variables lourdes (VH) et légères (VL) de l'anticorps A8**

| Anticorps monoclonal A8 | |
|---|---|
| VH-CDR1 (SEQ ID NO : 5) | GFTFSDFG |
| VH-CDR2 (SEQ ID NO : 6) | ISSGSSTR |
| VH-CDR3 (SEQ ID NO : 7) | ARSYDGYHVPSFAY |
| VL-CDR1 (SEQ ID NO : 8) | QNIVHSNGNTY |
| VL-CDR2 | RVS |
| VL-CDR3 (SEQ ID NO : 9) | FQGSHVPLT |

### 4/ Construction, production et caractérisation du scFv A8

### a/ Construction du fragment d'anticorps scFv A8

Le gène de synthèse du fragment variable simple chaîne (scFv) dérivé de l'anticorps A8 a été synthétisé par ATG:Biosynthetics GmbH (Allemagne). La séquence est conçue de la fusion des parties variables lourde et légère (SEQ ID NO : 1 / SEQ ID NO : 3) liées par une séquence codant pour le peptide (Gly₄Ser)₃ assurant la fonctionnalité de la protéine et terminés par une séquence codant le peptide His₆ (peptide HIS-tag) qui autorisera la purification du scFv. Afin de permettre l'insertion dans le plasmide d'expression, la séquence est flanquée par les sites enzymatiques de restriction PstI et SalI. Une séquence additionnelle est ajoutée entre l'extrémité 3' du VL et le site SalI autorisant la suppression du peptide His₆ si désiré. Les codons ont été optimisés pour l'expression en *E. coli.* Une représentation schématique de la construction du gène de synthèse est détaillée ci-dessous :

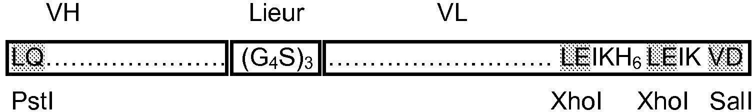

Le fragment d'anticorps a été inséré entre les sites enzymatiques PstI et XhoI du plasmide d'expression pSW1(ATG:Biosynthetics GmbH (Allemagne) selon E. S. Ward et collaborateurs (Ward et al., Nature, 341: 544-546, 1989) [4] qui contient sous le contrôle d'un promoteur inductible LacZ, une séquence signal PelB qui fusionnée en phase de lecture avec le gène du fragment d'anticorps recombinant, permet l'adressage de la protéine synthétisée vers le périplasme bactérien. Dans le périplasme, cette séquence signal est éliminée par une peptidase.

Après contrôle par séquençage de la qualité de la construction, le plasmide pSW1-scFv A8 a été transformé par choc thermique en bactéries HB2151 (T53040, Interchim, France) rendues chimiquement compétentes (Li et al., Afr. J. Biotechnol., 9(50) : 8549-8554, 2010) [5].

**Tableau 8 : Séquences nucléotidiques et peptidiques du scFv A8**

| scFv A8 | |
|---|---|
| Séquence nucléotidique (SEQ ID NO : 10) | |
| Séquence peptidique (SEQ ID NO : 11) | |

### b/ Production du fragment d'anticorps recombinant

### -Culture bactérienne

Une préculture est réalisée dans 5ml de milieu 2xYT contenant 50µg/ml d'ampicilline toute la nuit à 37°C. Le lendemain, 500µl de cette préculture est ensemencée dans 500ml du même milieu et mis à croître à 37°C à 150RPM jusqu'à obtenir une DO₆₀₀ₙₘ de 1,4. La synthèse du scFv est induite par l'ajout de 0,1mM d'IPTG 16h à 16°C à 15 RPM.

### -Extraction

Le milieu de culture est centrifugé 30min à 4500g à 4°C. La suite de la préparation se réalise à 4°C. Pour extraire le périplasme bactérien, le culot est remis en suspension et incubé dans 10ml de TES (Tris 0, M pH8, EDTA 0,5M, saccharose 0,5M) pendant 30min auxquels sont alors ajoutés 15ml de TES dilué au ¼ pour être de nouveau incubé 30min. L'extrait bactérien est centrifugé 30min à 10 000g. Le surnageant est mis à dialyser contre du PBS pendant une nuit. Le surnageant dialysé est traité immédiatement pour purifier le scFv ou conservé à -20°C jusqu'à utilisation.

La production du scFv dans le périplasme est analysée par Western blot avec anti His-Tag HRP (Réf. R93125 Life technologies, France).

### -Purification

Le périplasme est centrifugé pendant 20min à 5 000g à 4°C. Le surnageant est incubé avec HIS-Select® Nickel Affinity Gel (Ref P6611, Sigma-Aldrich, MO, USA) sous agitation pendant 1h à 4°C. Le gel est lavé avec un tampon phosphate de sodium 0,05M, NaCl 0,3M pH8 puis le même tampon additionné de 20mM d'imidazole jusqu'à obtenir une DO₂₈₀ₙₘ proche de 0. Le scFv est alors élué avec un tampon tampon phosphate de sodium 0,05M, NaCl 0,3M, 250mM imidazole pH8. L'éluat est dialysé contre du PBS toute la nuit. Il est conservé à -20°C.

### -Contrôle qualité

Le scFv purifié est analysé par électrophorèse sur gel de polyacrylamide à 15% après coloration au bleu de coomassie et par chromatographie d'exclusion sur colonne Sephadex™ 75 10/300 GL (Réf. 17-5174-01 GEHealthcare, Allemagne).

### 5/ Spécificité

La spécificité de l'anticorps A8 a été étudiée par différentes techniques : Western Blot, ELISA et résonance plasmonique de surface (SPR).

La spécificité du scFv A8 a été étudiée par ELISA.

Pour l'analyse conduite par Western Blot, 5µg de chaque hormone ou sous-unité ont été déposés sur gel de polyacrilamide 15% en conditions non-dénaturantes et en conditions dénaturantes. Après transfert sur nitrocellulose, l'anticorps A8 a été incubé à la concentration 5µg/ml et révélé avec un anticorps secondaire anti-IgG1 couplé à la peroxydase (HRP) (Réf. 115-035-205, Jackson ImmunoResearch Laboratories Inc) et un substrat cheluminescent, le West pico® (THERMO scientific, France).

Pour l'analyse de la spécificité conduite par ELISA, l'hormone étudiée a été préparée à la concentration de 10µg/ml dans du tampon carbonate de sodium 0,1M pH9,6 et distribuée à raison de 100µl par puits sur une plaque ELISA. Le temps d'adsorption a été de 18 heures à +4°C. Après cinq lavages, les puits sont traités avec 100µl de PBS additionné de Tween 0,1% et de BSA 1% pendant 45 min à 37°C, puis chaque anticorps ou scFv a été distribué à raison de 100µl/puits et incubés 1 heure à 37°C. Sur chaque hormone évaluée, les anticorps et les scFv ont été distribués à différentes concentrations selon une gamme de 10 à 250µg/ml pour les anticorps et de 10 à 150 ou 200µg/ml pour les scFv. Après cinq lavages, un anticorps secondaire anti-IgG1 couplé à la peroxydase (HRP) (Ref 115-035-205, Jackson ImmunoResearch Laboratories Inc) a été distribué à raison de 100µl/puits et incubé 1 heure à 37°C. Pour les scFv un anti-His Tag HRP (Ref R93125 Life technologies, France) a été utilisé. Après cinq lavages, l'activité enzymatique a été révélée avec du TMB distribué à raison de 100µl/puits. Le temps de révélation a été de 5 à 30 min à température ambiante selon la vitesse de la réaction. Après arrêt de la réaction avec H₂SO₄ 1M (50µl/puits) l'intensité de la réaction colorée (Densité Optique) est mesurée à l'aide d'un spectrophotomètre pour plaques ELISA.

Le pourcentage de réaction croisée a été calculé par rapport aux valeurs obtenues avec la FSH ovine (oFSH) considérée comme la valeur 100% de référence. Le pourcentage de réaction croisée a été calculé de façon classique en comparant les courbes dose-réponse obtenues avec la gamme de concentration de l'anticorps ou du scFv. A partir de la courbe obtenue avec l'hormone référence
- soit A la concentration donnant 50% de la densité optique maximale (ED 50). A partir de la courbe obtenue avec une autre hormone,
- soit B la concentration correspondante à la même valeur de densité optique que celle utilisée pour définir A .

Le pourcentage de réaction croisée est égal à A divisé par B et multiplié par 100 [(A/B) X 100].

Les analyses conduites par SPR ont été conduites sur un BiacoreT100 (GE Healthcare, France) et réalisées sur un anticorps de capture, anticorps anti-Fc d'IgG de souris, (Jackson laboratories, USA) préalablement immobilisé sur un sensorchip CM5 (GE Healthcare, France). L'anticorps A8 (20µg/ml) a été injecté pendant 45 secondes au débit de 10µl/min suivie d'une injection de l'hormone pendant 180 secondes au débit de 30 µl/min. après chaque séquence d'injections la surface est régénérée avec H₃PO₄ 100mM pendant 1 min. Le signal est ensuite soustrait des signaux non-spécifiques enregistré sur les pistes contrôles puis analysé par le logiciel BiaEval (version 2.03, GE Healthcare) du Biacore T100. Chaque concentration est injectée deux fois de façon aléatoire pour contrôler la répétabiité des résultats : la courbe de chaque réplicat est représentée sur les figures. Les calculs des constantes cinétiques sont réalisés sur l'ensemble des données.

### a/ Spécificité de A8 vis-à-vis des FSH et LH ovines et leurs sous-unités

La reconnaissance de A8 sur la oFSH dimérique, les sous-unités α et β oFSH, la oLH dimérique et la sous-unité β oLH a été d'abord étudiée par Western Blot. Seule la oFSH dimérique et la β oFSH ont été fortement reconnues à la fois en conditions non-dénaturantes et dénaturantes et ont donné un signal très important. Un très faible signal a été observé sur la oLH après un temps de révélation maximal. Aucun signal n'a été observé sur la β oLH dans les deux conditions. L'anticorps A8 reconnaît donc un épitope non-conformationnel situé sur βoFSH.

Par technique ELISA, la spécificité de l'anticorps A8 vis-à-vis de oFSH et de β oFSH a été confirmée.

Le tableau 9 illustre les pourcentages de réactions croisées de l'anticorps A8 avec les sous-unités (s.u.) α et β de la oFSH et la sous-unité β de la oLH

**Tableau 9**

| A8 | oFSH | s.u. α ovine | s.u. β oFSH | oLH | s.u. β oLH |
|---|---|---|---|---|---|
| Réaction croisée | 100% | 0% | 100% | 10% | 2% |

L'anticorps A8 ne reconnaît pas la sous-unité α ovine mais fortement la sous-unité β de la FSH ovine (100%) ; il croise très peu avec la oLH et β oLH. Sa spécificité est anti-sous unité β oFSH.

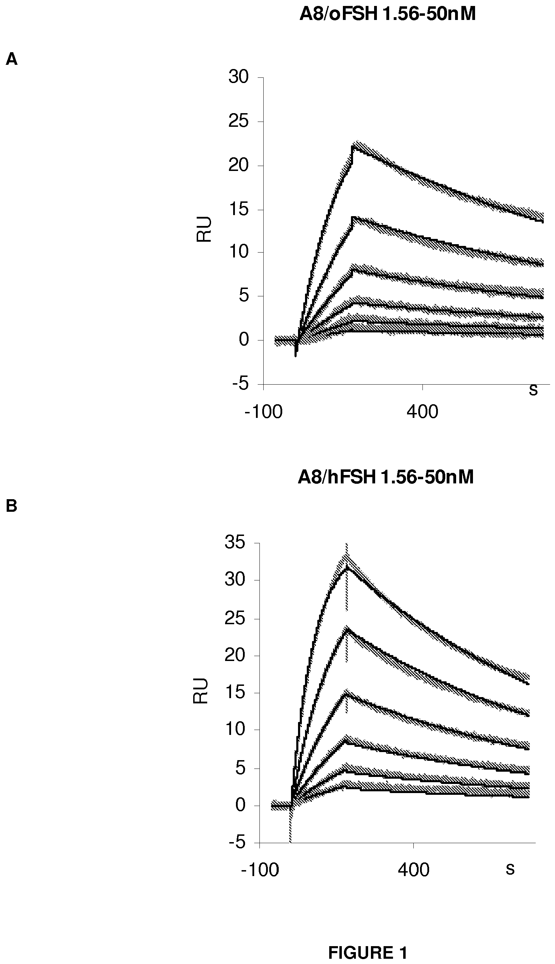

L'affinité de A8 pour la oFSH a été analysée par SPR sur un appareil Biacore T100® (GE Healthcare, France). La constante de dissociation, Kd, mesurée pour la oFSH est de 6,3.10⁻⁹M. La figure 1A illustre les profils d'interaction de la oFSH à différentes concentrations (1,56 à 50nM) sur l'anticorps A8 immobilisé. L'intensité d'interaction présente un Rmax de 34,1 unités de résonance (RU). Les constantes cinétiques sont de 1,3.10⁵±1,4.10² M⁻¹.s⁻¹ pour la vitesse d'association et de 8,2.10⁻⁴±8,4.10⁻⁷ s⁻¹ pour la vitesse de dissociation. La comparaison d'un profil d'interaction de la oFSH et de la oLH injectée à 100nM sur l'anticorps A8, donne un niveau de liaison spécifique de 23 RU et 4 RU respectivement, vingt secondes après la fin de l'injection, ce qui représente une réaction croisée de 15% de oLH par rapport à oFSH.

### b/ Spécificité de A8 vis-à-vis des FSH porcine et humaine

Le tableau 10 illustre les pourcentages de réactions croisées de A8 et du scFv A8 avec la FSH porcine (pFSH) et différentes FSH humaines :

**Tableau 10**

| | oFSH | pFSH | hFSH (Gonal F) | hFSH (Puregon) | hFSH (Fostimon) | hMG (Menopur) |
|---|---|---|---|---|---|---|
| A8 | 100% | <2% | 24% | 118% | 195% | 12% |

L'anticorps A8 ne présente pas de réaction croisée avec la FSH porcine à l'inverse des FSH humaines Puregon, Fostimon pour lequelles une forte liaison est enregistrée et, dans une moindre mesure, avec la Gonal-F et la hMG Menopur.

L'affinité de A8 pour la hFSH Gonal F a été analysée par SPR sur un appareil Biacore T100® (GE Healthcare, France). La constante de dissociation, Kd, mesurée pour la hFSH Gonal F est de 4,7.10⁻⁹M. La figure 1B illustre les profils d'interaction de la hFSH à différentes concentrations (1,56 à 50nM) sur l'anticorps A8 immobilisé. L'intensité d'interaction présente un Rmax de 38,5 unités de résonance (RU). Les constantes cinétiques sont de 2,4.10⁵ ± 2,1.10² M⁻¹.s⁻1 pour la vitesse d'association et de 1,1.10⁻³ ± 1.10⁻⁶ s⁻¹ pour la vitesse de dissociation. Les résultats obtenus sur Biacore confirment la reconnaissance spécifique de la FSH humaine Gonal F par l'anticorps A8 avec une bonne affinité. Ces résultats indiquent également que l'interaction hFSH Gonal F / A8 se fait mieux lorsque celle-ci est en phase liquide (Biacore) plutôt qu'adsorbée sur une phase solide (ELISA).

### c/ Spécificité de A8 vis-à-vis des choriogonadotropines équine et humaine

La reconnaissance spécifique de l'anticorps A8 a été étudiée par ELISA. Le signal obtenu après incubation de A8 à la concentration de 100µg/ml sur une eCC purifée au laboratoire, une eCG commerciale Chronogest® (MSD, France) et les hCG Chorulon et Endo 5000 adsorbées ont donné les valeurs de densité optique suivantes : 0,8 - 1,4 - 1,2 et 0,7 respectivement.

Les Kd ont été estimés pour la eCG Chronogest et les hCG Chorulon et Endo 5000 par ELISA. Dans ce cas, une estimation de la constante de dissociation Kd de l'anticorps A8 vis-à-vis des différentes hormones a été calculée sur GraphPad Prism (GraphPad Software Inc., San Diego, CA, USA, version 5) en utilisant la fonction "One site - Specific binding" dans un modèle de liaison à saturation ("saturation binding experiment model", GraphPad PRISM software). Les résultats sont illustrés dans le tableau 11.

**Tableau 11**

| A8 | eCG Chronogest | hCG Chorulon | hCG Endo 5000 |
|---|---|---|---|
| Kd (10⁻⁹ M) | 173,4 | 184 | 115,2 |

L'anticorps A8 présente donc une reconnaissance spécifique faible mais significative vis-à-vis des choriogonadotropines avec des valeurs de Kd de l'ordre du 0,1µM.

### d/ Spécificité du scFv A8

La spécificité du scFv A8 ainsi que l'estimation des Kd a été faite par ELISA selon la méthode décrite ci-dessus. La valeur des Kd estimés est illustrée dans les tableaux 12 et 13.

**Tableau 12**

| scFv A8 | oFSH | hFSH Gonal F | hFSH Puregon | hFSH Fostimon | hMG Menopur |
|---|---|---|---|---|---|
| Kd (10⁻⁶ M) | 3,12 | 4,62 | 3,21 | 3,28 | 4,22 |

Le scFv A8 reconnaît les FSH ovine et humaine avec un Kd de l'ordre du µM (entre 3,12 et 4,62) de même pour la hMG.

**Tableau 13**

| scFv A8 | eCG Chronogest | hCG Chorulon | hCG Endo 5000 |
|---|---|---|---|
| Kd (10⁻⁶ M) | 22,18 | 22,66 | 5,78 |

Le scFv A8 reconnaît également les eCG et hCG mais plus faiblement qie les FSH avec une valeur de Kd plus élevée (de 5,78 à 22,18µM).

### EXEMPLE 2 : MESURE IN VITRO DE L'EFFET POTENTIALISANT DU LIGAND DE L'INVENTION SUR LA BIOACTIVITE DE LA FSH

La mise en évidence de l'effet potentialisant des ligands de l'invention sur la bioactivité de la FSH a été réalisée en comparant la réponse biologique obtenue avec différentes types ou lignées cellulaires stimulées soit avec la FSH seule soit avec le complexe FSH / anticorps monoclonal (ACM).

Dans chacun des cas, la comparaison des courbes dose-réponse obtenues a permis de quantifier l'effet potentialisant *in vitro* de l'ACM sur l'activité biologique de la FSH complexée. L'analyse statistique des résultats a été faite par le logiciel Prism (GraphPad Software Inc., San Diego, CA, USA, version 5).

### 1/ Sur cultures primaires de cellules de granulosa bovines

L'effet potentialisant de l'AcM A8 sur la bioactivité de la FSH ovine (oFSH) a tout d'abord été caractérisé sur des cellules de granulosa bovines exprimant de façon endogène le récepteur FSH bovin.

Des surnageants d'hybridomes à la concentration finale de 0,1µg/ml d'anticorps A8 ont été incubés avec une gamme de FSH ovine ou humaine allant de 3ng/ml à 25ng/ml, 30mn à 37°C.

Les cellules de granulosa bovines ont été ponctionnées sur des ovaires de vache à partir de follicules de diamètre allant de 2 à 6 mm, selon le protocole décrit par Chopineau et al. (Mol. Cell Endocrinol., 92(2) : 229-39, 1993) [6] et Wehbi et al. (Endocrinology, 151(6) : 2788-2799, 2010) [7]. Les cellules de granulosa bovines en suspension dans un milieu McCoy's 5A (Lonza, Belgique, référence BE12-688F), préparées à raison de 80 000 cellules par 0,5ml, ont été stimulées pendant 3 heures à 37°C, sous agitation, en présence d'IBMX 48 µg/ml (Sigma Aldrich, France, référence I5879), par la FSH seule ou préalablement complexée à l'anticorps A8 selon le protocole ci-dessus. La réponse biologique mesurée a été la sécrétion en AMPc.

Après centrifugation, l'AMPc produit a été dosé dans le surnageant de culture à l'aide d'un kit ELISA (Biomedical Technologies Inc., MA, USA, BT-730).

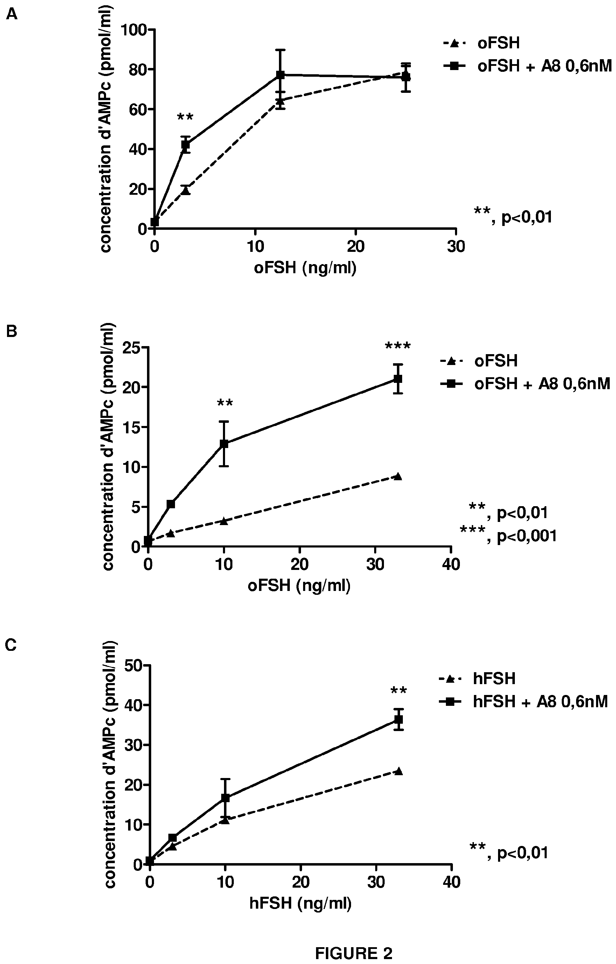

Les résultats présentés dans la figure 2A montrent une amplification de la sécrétion d'AMPc d'un facteur 2,1 fois pour la concentration 3ng/ml et d'un facteur 1,5 fois pour la concentration 12,5 ng/ml de la FSH ovine lorsque celle-ci est complexée à l'anticorps A8. L'analyse statistique par analyse de variances à deux variables (*two-way* ANOVA, GraphPad PRISM software) montre un effet potentialisant significatif de A8 sur la bioactivité de la oFSH (p<0,01) pour la concentration la plus basse (3,3ng/ml). Il n'y a pas de différence de stimulation à la concentration la plus élevée (25ng/ml).

### 2/ Sur lignée cellulaire HEK293 transfectées de façon stable avec le récepteur FSH humain

L'effet potentialisant de l'AcM A8 sur la FSH ovine et humaine a été mesuré sur des cellules HEK 293 exprimant de manière stable le récepteur FSH humain. Ce système a permis de mesurer la production d'AMPc suite à l'activation du récepteur de la FSH après une stimulation par la FSH seule ou par le complexe FSH / A8 pendant 1 heure à 37°C.

Pour cela, 60 000 cellules ont été réparties dans des puits de plaques 96 puits (Becton Dickinson, NJ, USA, référence 353072) et cultivées 24h à 37°C, 5% CO2 en atmosphère humide, dans 100 µl de milieu MEM (Ozyme, France, référence BE12-611F) contenant du SVF 10% (Lonza, Belgique, référence DE14-801F), pénicilline/streptomycine 1% (Sigma Aldrich, France, référence P-4333) et G418 400 µg/ml (Sigma Aldrich, France, référence A1720). Après 2h de sevrage dans du milieu MEM, les cellules ont été stimulées pendant 1h à 37°C. Le surnageant de culture a été récupéré et dosé à l'aide d'un kit ELISA (Biomedical Technologies Inc., MA, USA, BT-730). Les résultats expriment la quantité d'AMPc sécrétée en point final. Ils sont analysés grâce au logiciel Prism (GraphPad Software Inc., San Diego, CA, USA, version 5).

Les figures 2B et 2C illustrent l'effet potentialisant de l'anticorps A8 sur la bioactivité de la FSH ovine (oFSH) et de la FSH humaine (hFSH) *in vitro* sur des cellules HEK 293 transfectées de façon stable avec le récepteur FSH humain. Pour cela, les cellules ont été stimulées soit avec une gamme allant de 3ng/ml à 32,5ng/ml, soit avec les mêmes points de gamme FSH préalablement incubés, 30 minutes à 37°C, avec l'anticorps monoclonal (concentration finale 0,1µg/ml) avant la stimulation des cellules. Une analyse de variance à deux variables (*two-way* ANOVA, GraphPad PRISM software) a permis de comparer les courbes dose-réponse obtenues avec la FSH seule ou avec le complexe FSH/anticorps monoclonal.

L'anticorps A8 a montré un effet potentialisant important allant de 370% à 237% sur l'activité de la oFSH pour les concentrations 10ng/ml et 32,5ng/ml respectivement (figure 2B). Cet effet est significatif pour la concentration 10ng/ml de oFSH (p<0,01) et 32,5ng/ml (p<0,001).

Les résultats obtenus avec la FSH humaine recombinante (Gonal-F, laboratoire Serono) et illustrés sur la figure 2C montrent un effet potentialisant de l'activité hormonale de 150% à 10ng/ml de hFSH et de 156% pour la concentration 32,5ng/ml. Cet effet est significatif uniquement pour la concentration 32,5ng/ml de FSH humaine (p<0,01).

### 3/ Sur lignée cellulaire HEK293 transfectées de façon stable avec le récepteur FSH humain et avec le système Glosensor®

L'effet potentialisant de l'anticorps A8 sur la FSH a été mesuré en temps réel sur des cellules HEK 293 exprimant de manière stable le récepteur FSH humain et le vecteur GloSensor™ (Promega, France). Ce système cellulaire permet de suivre la production d'AMPc suite à la stimulation du récepteur FSH par l'agoniste (FSH seule ou complexe FSH / anticorps monoclonal) en temps réel. Suite à la liaison de l'AMPc sur la protéine GloSensor™, le substrat GloSensor™ (Promega, France, référence E1291) est hydrolysé et conduit à une émission de luminescence mesuré grâce à un lecteur PolarStar Optima (BMG Labtech, Allemagne) et exprimée en RLU (Relative Luminescence Unit). Cette lignée stable a été développée par l'équipe de Biologie et Bio Informatique des Systèmes de Signalisation au centre INRA Val de Loire, 37380 Nouzilly, (France) et a été mise gracieusement à disposition pour ces essais.

Les cellules HEK 293 ont été mises en culture à raison de 80 000 cellules par puits de microplaque 96 puits blanc à fond transparent (Dominique Dutscher, France, référence 655903) et cultivées dans 100µl de milieu MEM (Ozyme, France, référence BE12-611F) supplémenté de SVF 10% (Lonza, Belgique, référence DE14-801F), pénicilline / streptomycine 1% (Sigma Aldrich, France, référence P-4333), Hygromycine B 200µg/ml (Life Technologies™, France, référence 10687010) et G418 400µg/ml (Sigma Aldrich, France, référence A1720) pendant une nuit. Après 2h de sevrage dans 100µl de milieu MEM supplémenté de BSA 1% (PAA, France, référence K45012) et contenant 4% de substrat GloSensor™ pendant 2h à température ambiante à l'abri de la lumière, la plaque de cellule a été mise dans le lecteur PolarStar Optima et une première lecture a été faite pendant 5 minutes pour mesurer le niveau basal de luminescence. La plaque a ensuite été retirée du lecteur et 11µl de ligand (FSH seule ou complexe FSH / anticorps monoclonal) y ont été ajoutés de manière à obtenir les concentrations indiquées. La luminescence émise a ensuite été mesurée pendant environ 1h30.

Les résultats obtenus ont été analysés grâce au logiciel Prism (GraphPad Prism Software Inc., San Diego, CA, USA, version 5). La fonction non linéaire « log (agoniste) versus response » a été utilisée pour tracer la réponse en fonction de la concentration de FSH. Ceci a permis de caractériser et comparer l'EC50 pour la FSH seule et la FSH complexée à l'anticorps monoclonal. Pour chaque exemple, l'effet significatif du complexe FSH / anticorps potentialisant a été mesuré par analyse de variance à deux variables (*two-way* ANOVA, GraphPad PRISM software) en comparant les deux courbes dans leur totalité.

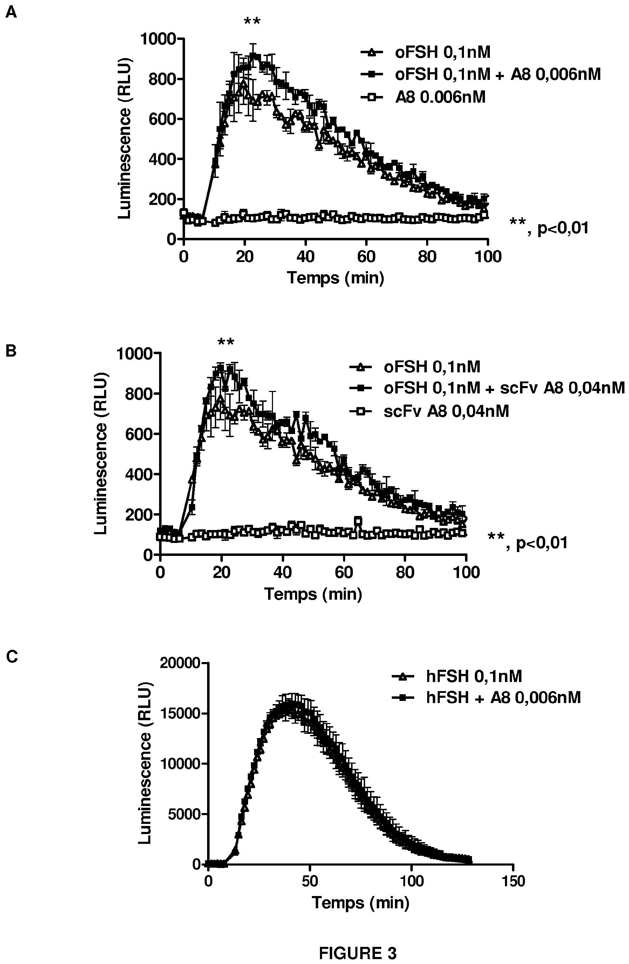

La figure 3A illustre la cinétique de production d'AMPc exprimée en unités relatives de luminescence en fonction du temps (en minutes) obtenues aux concentrations 0 et 0,1nM de FSH ovine seule ou complexée à l'anticorps A8 préparé à 0,006nM. On observe que les cellules « stimulées » par l'anticorps seul, sans FSH, ne montrent aucune réponse : le signal luminescent reste à son niveau basal. L'anticorps seul n'exerce donc aucun effet agoniste ou antagoniste sur le récepteur FSH humain exprimé par les cellules HEK 293. A l'inverse, l'anticorps A8 complexé à la oFSH amplifie de façon significative l'activité stimulante de l'hormone (p<0,01) : on observe une augmentation de la réponse maximale cellulaire de 130% passant de 740 RLU sous stimulation avec oFSH seule à 962 RLU sous stimulation avec le complexe oFSH/A8 à 25 min.

Le même effet potentialisant a été observé avec le scFv A8 (0,04nM) sur l'activité de la oFSH (0,1nM). Les résultats illustrés sur la figure 3B, montrent une augmentation de 137% de la réponse cellulaire maximale à 25min passant de 672 à 921 RLU avec le complexe oFSH/A8. Cet effet potentialisant bien que modéré est significatif (p<0,01). Tout comme l'anticorps entier A8, le scFv seul n'a aucun effet sur la réponse cellulaire.

Enfin, l'effet de l'anticorps A8 (0,006nM) a été évalué sur la bioactivité de la hFSH Gonal F (0,1nM). Les résultats illustrés sur la figure 3C ne montrent pas de différence significative entre la réponse des cellules stimulées par la hFSH seule ou par le complexe hFSH/A8.

### EXEMPLE 3 : MESURE IN VIVO DE L'EFFET POTENTIALISANT DU LIGAND DE L'INVENTION SUR LA BIOACTIVITE DES FSH et LH/CG dans le modèle Rat

Après avoir été caractérisé *in vitro*, l'effet potentialisant de l'anticorps monoclonal A8 a été caractérisé *in vivo*, chez le rat femelle pour son effet sur la bioactivité de la FSH et chez le rat mâle pour son effet sur la bioactivité des hCG et eCG qu'il reconnaît également.

Pour mesurer la bioactivité FSH, le protocole utilisé a été celui du dosage biologique décrit par Steelman et Pohley (Steelman SL, Pohley FM. Endocrinology, 53 :604-616. 1953) [8].

Pour mesurer la bioactivité LH, le protocole utilisé a été celui du dosage biologique décrit par Scobey et collaborateurs (Scobey et al, Reprod. Biol. Endocr. 3 :61, 2005) [9].

La bioactivité de la eCG a été mesurée en utilisant le dosage biologique décrit par Cole et Erway (Cole HH, Erway J. Endocrinology, 29 :514. 1941) [10].

L'analyse statistique a été réalisée avec le logiciel GraphPad Prism (GraphPad Software Inc., San Diego, CA, USA, version 5). Les résultats portant sur des expériences réalisées sur des lots de 5 animaux, une analyse de variance à une variable, non paramétrique (test de Kruskal Wallis), suivie d'une correction de Dunns, a été appliquée ou un test-t non-paramétrique (test de Mann-Whitney). Pour les résultats portant sur des effectifs plus importants (n>30) issus de la compilation de plusieurs bioassays, un test paramétrique (test t de Student non apparié) suivi d'une correction de Bonferroni a été appliqué.

### Effet potentialisant des anticorps sur la bioactivité de la FSH chez la Ratte

L'effet potentialisant de l'anticorps A8 et de son scFv a été étudié sur la FSH ovine et sur une FSH humaine utilisée en reproduction humaine dans le cadre des traitements de procréation médicalement assistée, la Gonal-F, FSH recombinante commercialisée par Merck Serono.

Comme décrit dans le protocole de Steelman et Pohley, des rattes immatures de 21 jours ont reçu pendant trois jours consécutifs 2 injections matin et soir, de 100µl d'un mélange de hCG et FSH comportant une quantité constante de hCG (3,5UI) additionnée d'une quantité variable de FSH allant de 0,5 à 1,5 UI pour la FSH humaine Gonal F ou de 0,5 à 2 µg pour la FSH ovine. Les injections ont été réalisées par voie sous-cutanée au niveau de la nuque. Chaque expérience comprenait au minimum 4 lots : un lot traité avec du sérum physiologique (sérum Φ), un lot traité avec l'anticorps ou le scFv seul, un lot traité avec le mélange hCG+FSH, un lot traité avec le mélange hCG/FSH additionné de 2µg d'anticorps ou de scFv purifié.

Dans le cas d'un traitement avec le complexe hormone/anticorps ou scFv, avant l'injection, le mélange FSH + anticorps a été préalablement incubé 20min à 37°C ou à température ambiante indifféremment, puis ajouté à la hCG. La hCG peut indifféremment être mélangée à la FSH pendant l'incubation du complexe.

Le quatrième jour, les rattes ont été pesées, et leurs ovaires ont été prélevés, disséqués puis pesés. Les résultats sont exprimés en milligramme d'ovaire/100 grammes de poids corporel. L'augmentation du poids des ovaires est proportionnelle à la quantité de FSH bioactive injectée. Ceci permet de quantifier et de comparer la bioactivité d'une même quantité d'hormone injectée seule ou en complexe avec l'anticorps A8.

La comparaison des deux réponses a ainsi permis de quantifier l'effet potentialisant de l'anticorps A8 ou de son scFv.

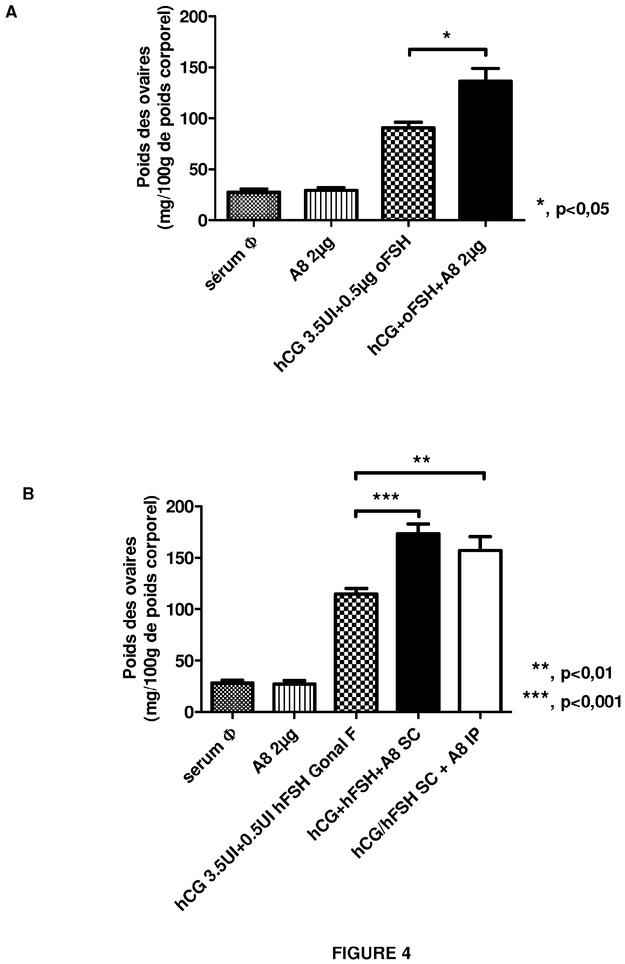

La figure 4A illustre un exemple représentatif de l'effet de l'anticorps A8 sur la bioactivité FSH ovine. Chaque lot comportait 5 femelles. Le lot traité avec l'anticorps A8 injecté seul a présenté le même poids moyen des ovaires que les femelles du lot contrôle ayant reçu du sérum physiologique (28,6 mg et 31mg respectivement). Le lot ayant reçu le traitement hormonal classique (hCG 3,5UI +oFSH 0,5µg) a donné un poids moyen des ovaires de 90,5 ± 6mg. Le lot traité avec la FSH ovine préalablement complexée à l'anticorps A8 a présenté un poids moyen de 136 ± 8 mg soit une augmentation significative de la bioactivité FSH de 150% par rapport au lot ayant reçu le traitement hormonal classique (p<0.05, test de Mann-Whitney).

L'effet de l'anticorps A8 sur la FSH humaine Gonal F a également été analysé. Les résultats sont présentés figure 4B. Le lot de femelles (n=11) traité avec le mélange hormonal classique (hCG 3,5UI +oFSH 0,5µg) a donné un poids moyen des ovaires de 114 ± 6mg. Le lot traité avec la FSH humaine préalablement complexée à l'anticorps A8 + hCG (n=10) a présenté un poids moyen de 173,5 ± 8mg soit une augmentation significative de la bioactivité FSH de 152% par rapport au lot ayant reçu le traitement hormonal classique (p<0.001, one-way anova).

Afin d'évaluer la capacité de l'anticorps à potentialiser la FSH en étant injecté de façon différée par rapport à l'hormone, le même traitement a été réalisé en injectant l'anticorps A8 par voie intra-péritonéale 15 minutes avant l'injection du mélange hCG+hFSH par voie sous-cutanée classique. Le résultat obtenu est illustré sur la figure 4B et montre une augmentation significative du poids des ovaires (p<0,01) de 138% avec un poids moyen de 157 ± 14mg.

Ces résultats démontrent d'une part que l'anticorps A8 peut exercer un effet potentialisant de 150% sur hFSH en étant injecté en complexe avec l'hormone ou en étant injecté seul et de façon indépendante de l'hormone. Dans ce dernier cas, A8 a complexé la FSH in vivo dans la circulation après son injection et a potentialisé la bioactivité de l'hormone. L'effet potentialisant *in vivo* de l'anticorps n'est pas dépendant de l'injection d'un complexe AC/hormone.

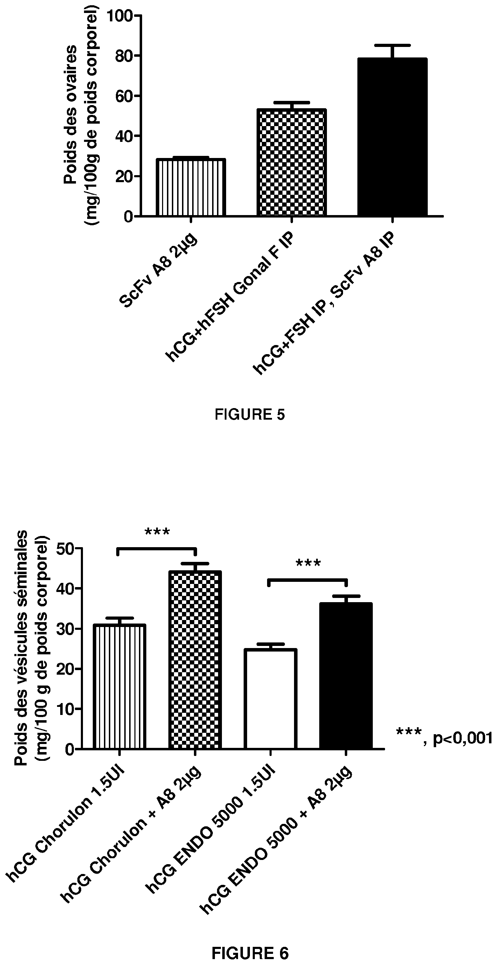

Un même type d'expérience a été réalisé afin d'évaluer l'effet du scFv A8 sur la bioactivité de la hFSH *in vivo* comparativement à l'anticorps entier. Seul un mode d'injection par voie intra-péritonéale a permis d'observer un effet potentialisant du scFv. Les résultats illustrés figure 5 montrent qu'une augmentation de 276% a été obtenue chez le lot de femelles (n=6) traitées avec le complexe scFv A8/hFSH avec un poids moyen des ovaires de 78,2 ± 8mg contre 28,3 ± 2mg chez le lot contrôle ; cet effet n'est cependant pas significatif. Le poids moyen des ovaires obtenu chez le lot traité avec le complexe A8/hFSH a été de 52,8 ± 4mg soit une augmentation de 186%, non significative.

### Effet potentialisant de l'anticorps A8 sur la bioactivité de la hCG chez le Rat

L'effet de l'anticorps a été étudié sur deux préparations de hCG extraites, l'une utilisée en reproduction humaine dans le cadre des traitements de procréation médicalement assistée : l'ENDO 5000 (laboratoire Schering-Plough) et l'autre utilisée en médecine vétérinaire : la Chorulon (laboratoire MSD).

Dans le protocole de Scobey et collaborateurs [9], la bioactivité de la LH ou de la hCG est quantifiée par rapport à l'augmentation du poids des vésicules séminales dont le développement est androgéno-dépendant. Le poids varie proportionnellement à l'activité de la hCG et permet donc de quantifier et comparer l'activité biologique de l'hormone injectée seule ou complexée avec l'anticorps étudié. Le protocole a été réalisé avec des ratons de 25 jours qui ont été injectés par voie sous-cutanée, une fois par jour pendant quatre jours avec 100µl de 1,5UI de hCG ou d'un mélange 1,5UI hCG + 2µg d'anticorps préalablement incubé 20mn à 37°C. Le cinquième jour, les rats ont été pesés puis sacrifiés. Leurs vésicules séminales ont été prélevées, disséquées et pesées. Le poids des vésicules séminales est exprimé en mg/100 grammes de poids corporel afin de pouvoir comparer et pooler les résultats obtenus avec différents lots. Dans chaque expérience, chacune des conditions a été testée sur un lot de 5 rats. Une même expérience a été répétée plusieurs fois.

L'effet potentialisant de A8 a été évalué sur la hCG Chorulon et la hCG Endo 5000. Les résultats sont illustrés sur la figure 6 et sont issus de la compilation de plusieurs expériences. L'effectif de chaque lot expérimental était ainsi de 12 animaux.

Un effet potentialisant significatif (p<0,001, test de Mann-Whitney) a été obtenu avec le complexe hCG Chorulon / A8 avec une augmentation de 145% du poids des vésicules séminales par rapport au lot traité avec hCG seule: 44,11 ± 2mg contre 30,5 ± 2mg respectivement. Un même effet significatif (p<0,001, test de Mann-Whitney) a été obtenu avec le complexe hCG Endo 5000 / A8 traduit par une augmentation de 146% du poids des vésicules séminales par rapport au lot traité avec hCG seule: 36,2 ± 2,4mg / 100g de poids corporel contre 24,71 ± 3,1mg respectivement.

### Effet potentialisant de l'anticorps A8 sur la bioactivité de la eCG chez la ratte

L'effet de l'anticorps A8 a été étudié sur la eCG Chronogest® *in vivo* chez la ratte.

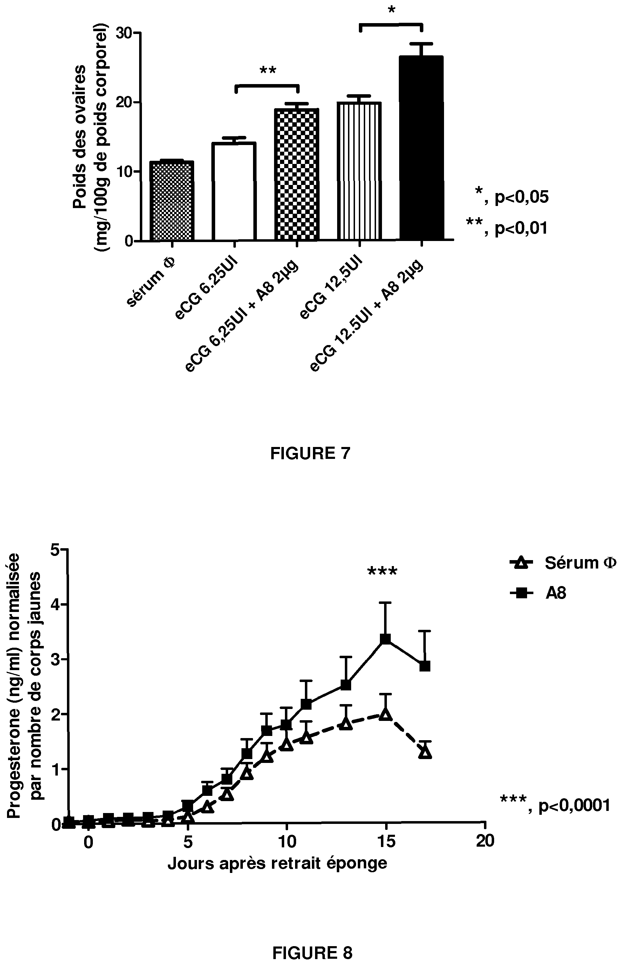

Pour cela, la bioactivité de la eCG a été mesurée en utilisant le dosage biologique décrit par Cole et Erway (Cole HH, Erway J. Endocrinology, 29 :514. 1941) [10]. Dans ce protocole, la bioactivité de eCG a été quantifiée chez des rattes immatures de 21 jours qui ont reçu une injection de eCG de 6,25UI ou de 12,5UI administrée dans 100µl par voie sous-cutanée au niveau de la nuque. Quarante huit heures plus tard, les rattes ont été pesées, et leurs ovaires ont été prélevés, disséqués puis pesés. Les résultats sont exprimés en milligramme d'ovaire/100 grammes de poids corporel. L'augmentation du poids des ovaires est proportionnelle à la quantité de eCG bioactive injectée. Ceci permet de quantifier et de comparer la bioactivité d'une même quantité d'hormone injectée seule ou en complexe avec un anticorps. Chaque expérience comprenait au minimum 5 lots: un lot traité avec du sérum physiologique (sérum Φ), un lot traité avec eCG 6,25 UI seule, un lot traité avec eCG 6,25UI additionnée de 2µg d'anticorps A8, un lot traité avec eCG 12,5UI seule et un lot traité avec eCG 12,5UI additionné de 2µg d'anticorps A8. Chaque lot comportait 5 à 6 femelles. Les résultats obtenus sont illustrés par la figure 7. Un effet potentialisant significatif (p<0,01, test de Mann Whitney) a été obtenu avec le complexe A8 / eCG 6,25UI montrant une augmentation de 134% du poids des ovaires / 100g de poids corporel par rapport au lot traité avec eCG seule: 18,78 ± 2mg contre 14 ± 1,8mg respectivement. De même, un effet potentialisant significatif (p<0,05, test de Mann Whitney) a été observé avec le complexe A8 / eCG 12,5UI avec une augmentation de 133% du poids des ovaires par rapport au lot traité avec eCG 12,5UI seule: 26,3 ± 3mg contre 19,72 ± 1,8mg respectivement.

En conclusion, ces expériences démontrent que l'anticorps A8 a la capacité de potentialiser significativement l'activité de la FSH, de la hCG et de la eCG *in vivo* chez le rat mâle ou femelle.

### EXEMPLE 4: MESURE IN VIVO DE L'EFFET POTENTIALISANT DU LIGAND DE L'INVENTION SUR LA BIOACTIVITE DES GONADOTROPINES ENDOGENES CHEZ LA BREBIS

Après avoir démontré et caractérisé l'effet potentialisant *in vivo*, de anticorps monoclonal A8 chez un rongeur (animal de petite taille), l'objectif a été d'étudier l'effet de l'anticorps sur l'activité de la FSH chez un animal de rente, de plus grande taille : la brebis.

Pour cela, une étude a été réalisée sur des brebis Ile de France, pubères, toutes du même âge, dans le but d'évaluer l'effet potentialisant des anticorps sur les propres hormones des brebis traitées (hormones endogènes). L'étude de la spécificité a montré en effet une forte liaison de l'anticorps A8 pour la FSH ovine. Dans cet objectif, un traitement ne comprenant que l'injection de l'anticorps seul a été mis au point pour en évaluer son efficacité. Il a été comparé dans une seconde expérience à un traiteemnt comportant l'injection du complexe FSH / anticorps A8.

L'effet potentialisant de l'anticorps A8 a été évalué au cours de protocoles réalisés en saison sexuelle (octobre et janvier). Les protocoles ont tous été réalisés sur des brebis dont le cycle ovulatoire a été préalablement synchronisé par la pose d'une éponge vaginale imprégnée d'un progestagène (45 mg d'acétate de fluorogestone (FGA) - MSD) pendant 14 jours. L'effet potentialisant a été analysé en comparant la réponse ovulatoire (nombre d'ovulations) et la mise en place d'un ou de plusieurs corps jaunes fonctionnels de bonne qualité (amplitude de la sécrétion de progestérone) chez des brebis contrôles (lot sérum physiologique), des brebis stimulées par un traitement de FSH porcine (lot FSH), des brebis stimulées par un anticorps seul (lot anticorps) et, dans un des protocoles, des brebis traitées par le complexe anticorps A8 / pFSH.

Dans chaque protocole, un dosage de la LH plasmatique a été réalisé par méthode ELISA afin de détecter et dater le pic pré-ovulatoire de LH. Pour évaluer la réponse ovulatoire, une observation endoscopique des ovaires a été réalisée par laparoscopie, sous anesthésie, huit jours après le retrait de l'éponge vaginale, afin de compter le nombre de corps jaunes et observer leur aspect.

Pour évaluer la fonctionnalité et la qualité du ou des corps jaunes, un dosage ELISA quantitatif de progestérone a été réalisé à partir de prises de sang quotidiennes faites du premier au 21^{ème} jour après le retrait de l'éponge.

Toutes les analyses statistiques ont été faites avec le logiciel GraphPad Prism Version 5.0 (GraphPad, San Diego, CA, USA).

L'effet potentialisant de l'anticorps A8 (IgG1) a été évalué dans deux protocoles (1 et 2).

### Le protocole 1, réalisé en janvier, comprenait deux lots :

- un lot "anticorps A8" (n=8) a reçu une injection de A8 purifié par voie intra-musculaire : 2 mg 24H avant le retrait de l'éponge
- un lot "contrôle" (n=8) a reçu une injection de sérum physiologique, par voie intramusculaire, 24h avant le retrait de l'éponge

L'analyse de la réponse ovulatoire a donné les résultats présentés dans le tableau 14 ci-dessous.

**Tableau 14**

| | Lot sérum Φ | Lot A8 seul | Statistiques |
|---|---|---|---|
| Nombre de brebis par lot | 8 | 8 | |
| Nombre de brebis ayant ovulé par lot | 7/8 (88%) | 7/8 (88%) | NS |
| Nombre de corps jaunes par brebis ayant ovulé | 1,5 ± 0,7 | 1,25 ± 0,7 | NS |
| Moment du pic de LH (heures après retrait) | 57,75 ± 7 | 59,25 ± 5 | NS |

L'analyse statistique a été faite par un test exact de Fisher.

Les résultats obtenus dans le lot A8 ne montrent pas d'effet significatif sur la réponse ovulatoire par rapport au lot contrôle. Aucun des paramètres mesurés ne présentent de tendance.

Le profil de sécrétion de la progestérone au cours de la phase lutéale obtenue dans les trois lots, a ensuite été analysé. Pour chaque individu, les valeurs de concentration en progestérone (ng/ml) ont été normalisées par nombre de corps jaunes. Chaque courbe représente la moyenne des valeurs de progestérone obtenue chez les femelles de chaque lot à un temps t.

Les résultats illustrés sur la figure 8 montrent un effet potentialisant significatif de l'anticorps A8 sur l'intensité de la sécrétion de progestérone par corps jaune et sur le début de sa mise en place (p<0,0001, test de Wilcoxon : test non paramétrique apparié). La courbe obtenue avec le lot A8 indique un début de sécrétion de progestérone dès J4 contrairement à la courbe du lot contrôle indiquant une mise en place de la sécrétion de progestérone après J5. L'augmentation significative de la sécrétion de progestérone obtenue dans le lot A8 se maintient tout au long de la phase lutéale jusqu'en fin de cycle. Les valeurs moyennes de progestérone à J10 sont de 1,8 ng/ml et 1,5 ng/ml respectivement pour les lots A8 et sérum Φ et de 3,7 et 2,8 ng/ml à J15.

L'anticorps A8 injecté seul est donc capable de potentialiser l'activité des gonadotropines endogènes chez la brebis.

### Le protocole 2, réalisé en octobre comportait 4 lots :

- le lot "anticorps A8" (n=6) a reçu une seule injection par voie intra-musculaire de 2 mg d'anticorps A8 24h avant retrait de l'éponge.
- le lot "contrôle" (n=5) a reçu une injection de sérum physiologique, par voie intramusculaire, 24h avant le retrait de l'éponge
- le lot "FSH" (n=5) a reçu une injection par voie intra-musculaire de 100µg de FSH porcine (pFSH) 24h avant le retrait de l'éponge et de 90 µg 12h avant le retrait de l'éponge.
- le lot "FSH + A8" (n=6) a reçu une injection par voie intra-musculaire du complexe pFSH / A8 (100µg de pFSH préincubés avec A8 2mg), 24h avant le retrait de l'éponge. L'analyse de la réponse ovulatoire a donné les résultats présentés dans le tableau 15 ci-dessous.

**Tableau 15**

| | Lot sérum Φ | Lot FSH | Lot A8 seul | Lot FSH+A8 |
|---|---|---|---|---|
| Nombre de brebis par lot | 5 | 5 | 6 | 6 |
| Nombre de brebis ayant ovulé par lot | 5/5 | 4/5 | 6/6 | 6/6 |
| Nombre de corps jaunes par brebis du lot | 2,6 ± 0,3 | 5,4 ± 4,4 | 2,2 ± 0,2 | 5,5 ± 3,2 |
| Nombre de corps jaunes par brebis ayant ovulé | 2,6 ± 0,3 | 6,75 ± 5,6 | 2,2 ± 0,2 | 5,5 ± 3,2 |
| Moment du pic de LH (heures après retrait) | 61,2 ± 6,8 | 69 ± 12,4 | 56 ± 4,4 | 58 ± 5,3 |

L'analyse statistique de ces différents paramètres n'a pas mis en évidence de différence significative entre les différents lots notamment entre le lot A8 et le lot FSH ou FSH+A8.

Le moment moyen d'apparition du pic de LH n'est pas significativement différent entre les trois lots. On observe malgré tout une tendance à moins de variabilité dans la venue du pic de LH (donc du moment de l'ovulation) dans le lot A8 et A8+FSH par rapport au lot FSH et surtout sérum Φ.

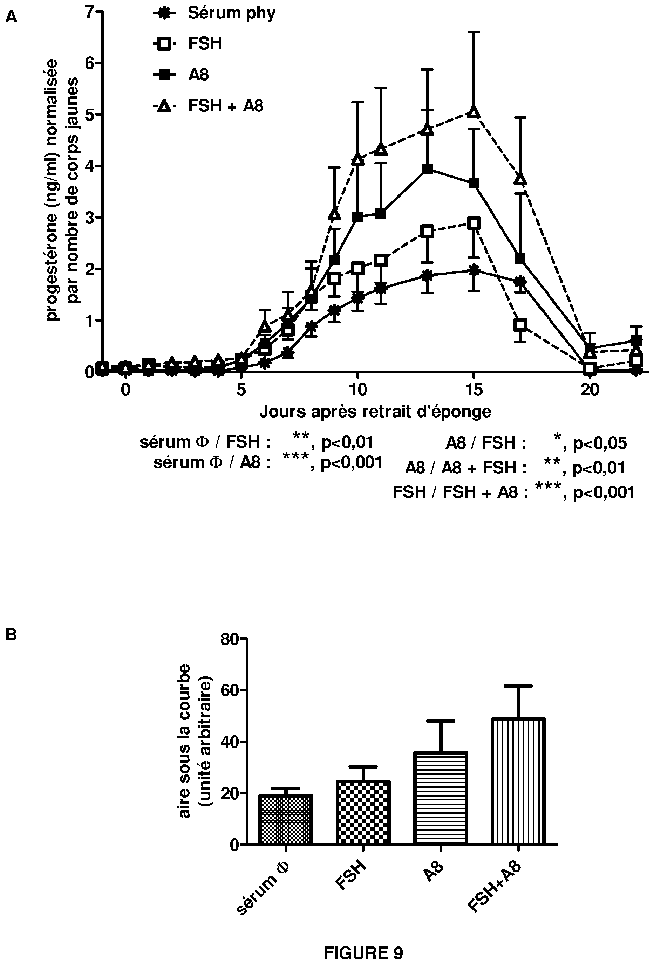

Le profil de sécrétion de la progestérone au cours de la phase lutéale suite à l'ovulation, est illustré dans la figure 9A. Pour chaque individu, les valeurs de concentration en progestérone (ng/ml) ont été normalisées par nombre de corps jaunes. Chaque courbe de la figure représente la moyenne des valeurs de progestérone obtenue chez les femelles de chaque lot. Un effet potentialisant significatif de l'anticorps A8 a été observé sur l'intensité de la sécrétion de progestérone par corps jaune se traduisant par une augmentation tout au long de la phase lutéale par rapport aux courbes du lot FSH (p<0,05 ; test de Wilcoxon) et du lot sérum Φ (p<0,001). Les valeurs moyennes à J10 sont de 3ng/ml, 2,1 et 1,42 ng/ml respectivement pour les lots A8, FSH et sérum Φ, et de 3,66 - 2,88 et 1,97 ng/ml à J15. Le lot A8+FSH est significativement différent du lot A8 (p<0,01) avec une valeur moyenne de progestérone de 4,13ng/ml à J10 et 5,05ng/ml à J15.

Un calcul d'aire sous la courbe (AUC) a été réalisé avec le logiciel GraphPad Prism version 5.0. Les résultats sont illustrés sur la figure 9B. Une analyse statistique par le test t non paramétrique de Mann-Whitney n'a pas mis en évidence de différence significative entre les lots malgré une tendance à une augmentation de l'AUC pour les lots A8 (35,7±12) et FSH+A8 (48,73±13) comparativement au lot sérum Φ (18,87±2) et FSH (24,4±5).

En conclusion, l'utilisation de l'anticorps A8 seul ou en complexe avec pFSH sous forme d'une seule injection intra-musculaire de 2mg a donné de façon significative de meilleurs résultats en termes de délai de mise en place (J4) et de intensité de sécrétion de la progestérone très supérieure à celle observée suite à un traitement avec pFSH. Il est à souligner que cette propriété potetialisante de A8 par rapport à un traitement FSH est importante. En effet, la concentration plasmatique de progestérone est un facteur clé du développement embryonnaire, particulièrement dans ses phases précoces.

### EXEMPLE 5: PREDICTION DE L'EPITOPE RECONNU PAR LE LIGAND A8 DE L'INVENTION, ET PREDICTION DE SON PARATOPE.

L'épitope respectif de l'anticorps A8 a été déterminé sur les hormones gonadotropes de différentes espèces en utilisant un algorithme d'amarrage des protéines basé sur une modélisation de la structure des protéines par un diagramme de Voronoï et une optimisation par différentes méthodes d'apprentissages évolutionnaires de fonctions de score permettant de différencier les conformations natives et non-natives (Bernauer et al., Bioinformatics 2007, 5:555) [14], (Bernauer et al., Bioinformatics 2008, 24:652) [15], (Bourquard et al., PLoS One 2011, 6:e18541) [16] et (Bourquard et al., Sci. Reports 2015, 5 :10760) [17].

L'anticorps a été amarré avec la FSH humaine (hFSH), la LH humaine (hLH), la CG humaine (hCG), la FSH ovine (oFSH) et la LH ovine (oLH), la FSH porcine (pFSH) et la LH porcine (pLH). Les structures cristallographiques de la hFSH et de la hCG sont disponibles dans la Protein Data Bank (PDB) : 4MQW et 1QFW respectivement. La structure de la FSH humaine complexée avec le domaine extracellulaire du récepteur FSH humain a été utilisé (Fan et Hendrickson, Nature 2005, 433:269) [18]. Pour les autres hormones (hLH, oFSH, oLH, pFSH et pLH), des modèles par homologie ont été réalisés puis utilisés pour l'amarrage.

La structure 3D de l'anticorps A8 n'étant pas disponible, l'étude a été faite à partir des séquences des fragments monovalents VH et VL de A8. Pour cela, des modèles par homologie des parties variables ont été réalisés. Les modèles des VH et VL ont été réalisés séparément, à partir de structures différentes, et leur orientation relative a été déterminée à partir de la structure ayant servi de support pour la modélisation du VH. Les structures utilisées pour les modèles par homologie sont disponibles dans la Protein Data Bank (PDB) : 2AAB pour le VH de A8 et 3IFP pour le VL de A8.

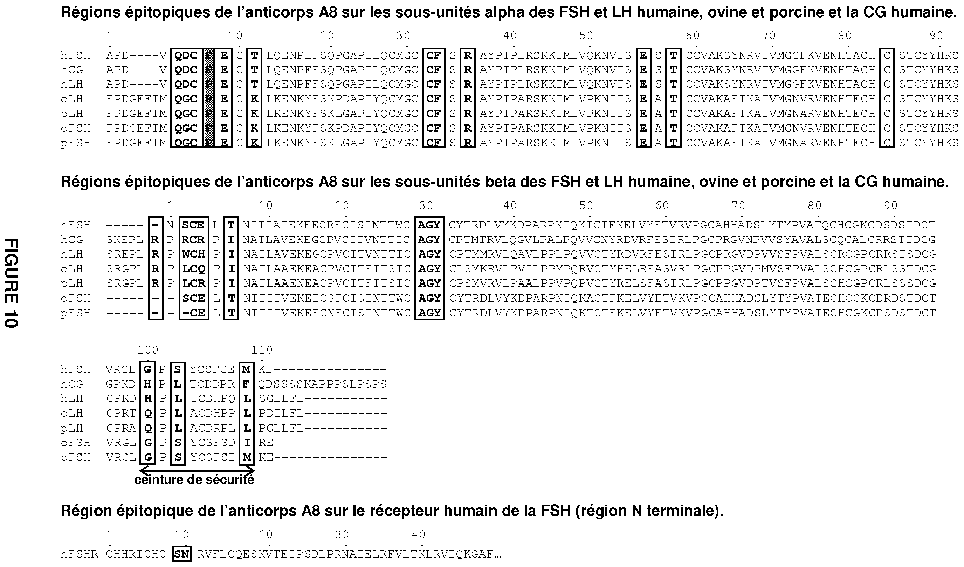

Les résultats d'amarrage sont illustrés sur la Figure 10. Il apparaît que le ligand A8 s'amarre de manière similaire sur les sept hormones cibles. L'épitope est défini par plusieurs régions situées de façon discontinue sur les sous-unités alpha et béta de la FSH et concerne également deux résidus contigus de l'extrémité N-terminale du récepteur FSH humain. L'épitope du ligand A8 est donc conformationnel : il est constitué de plusieurs régions séquentielles de l'hormone et de deux résidus dans l'extrémité N-terminale du récepteur FSH. L'ensemble de ces régions se trouvent spatialement proches dans la conformation native de l'hormone et du récepteur activé.

Les différents résidus de l'hormone et du récepteur impliqués dans l'interface avec l'anticorps A8 sont entourés par des rectangles sur la Figure 10. Parmi eux, les trois résidus notés en grisé sur la sous-unité alpha, Pro8, Glu56 et Thr58, sont impliqués dans l'interaction principale et ont un rôle majeur dans la reconnaissance anticorps/antigène. Parmi les autres résidus impliqués dans l'interface, certains se situent sur la sous-unité alpha dans une première région (Gln5-Asp6-Cys7, Glu9, Thr11), une deuxième région (Cys32-Phe33 et Arg35) et en position 84 (Cys84). Sur la sous-unité béta, les résidus sont localisés en trois régions : Ser2-Cys3-Glu4 et Thr6, Ala29-Gly30-Tyr31 et sur l'extrémité C-terminale Gly100, Ser102 et Met109 . Ces trois derniers résidus sont localisés dans la région appelée « ceinture de sécurité » ("seat belt") dont le rôle est de stabiliser l'association du dimère alpha/béta de l'hormone. Il semble donc que la liaison du ligand A8 dans cette région sécurise la fermeture de la ceinture de sécurité et contribue ainsi à la stabilité du dimère, indispensable à la bioactivité de l'hormone. De plus le ligand A8 reconnaît de façon majeure le résidu Glu56 sur la sous-unité alpha, impliqué dans la fixation et le maintien de la "ceinture de sécurité" autour de la sous-unité alpha.

L'épitope de l'anticorps A8 comprend également deux résidus de la région N-terminale du récepteur FSH humain : Ser9 et Asn10.

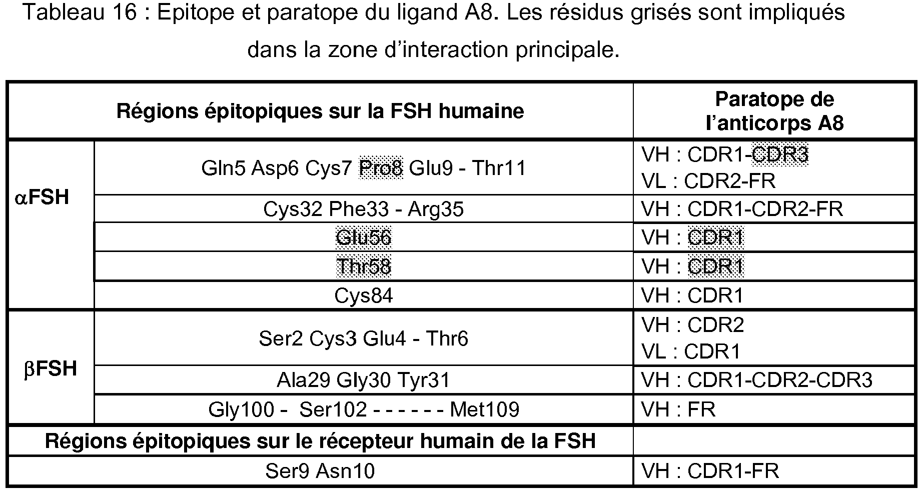

Le tableau 16, illustre les différentes régions constituant l'épitope du ligand A8 et celles constituant son paratope. La localisation indiquée pour les résidus des chaînes VH et VL sont celles obtenues sur la base IMGT tel que décrit dans l'exemple 1 de la présente invention.

L'hormone est essentiellement reconnue par les CDR1, CDR2 et CDR3 et certains résidus du framework 3 de la chaîne VH. Les CDR1, CDR2 et framework 3 de la chaîne VL sont également impliqués.

Les résidus du paratope impliqués dans l'interaction principale sont les résidus Tyr103 dans le CDR3 de VH, et Ser30 et Thr28 dans le CDR1 de VH. Les trois résidus localisés sur la ceinture de sécurité sont reconnus par les résidus Asp74, Pro75 et Lys76 du framework 3 de la chaîne VH.

Les deux résidus de la région N-terminale du récepteur FSH humain, Ser9 et Asn10, interagissent respectivement avec le résidu Glu1 du framework 1 de VH et le résidu Gly26 du CDR1 de VH. Seule VH intergit avec cette région.

En conclusion, le ligand A8 reconnaît un épitope conformationel impliquant essentiellement la sous-unité alpha, mais également la sous-unité béta de la hFSH et son extrémité C-terminale (ceinture de sécurité). Il reconnaît également deux résidus de l'ectodomaine du récepteur FSH. L'épitope conformationnel du ligand A8 lui permet de stabiliser l'association du dimère de l'hormone et de stabiliser la liaison de l'hormone sur son récepteur. Ces deux mécanismes sont complémentaires et pourraient aboutir à une meilleure interaction de l'hormone sur son récepteur et à une meilleure efficacité de celle-ci.

### Listes des références

1. Brevet EP 1518863
2. Demande Internationale WO 2012/066519
3. Corpet, Nucl. Acids Res., 16(22) : 10881-10890, 1988
4. Ward et al. Nature, 341 : 544-546, 1989)
5. Li et al., Afr. J. Biotechnol., 9(50) : 8549-8554, 2010
6. Chopineau et al., Mol. Cell Endocrinol., 92(2) : 229-239, 1993
7. Wehbi et al., Endocrinology, 151(6) : 2788-2799, 2010
8. Reverchon et al., Human Reprod., 27(6) : 1790-1800, 2012
9. Steelman SL, Pohley FM., Endocrinology, 53 :604-616, 1953
10. Scobey et al, Reprod. Biol. Endocr. 3 :61, 2005
11. Cole, H. H. and J. Erway. Endocrinology 29:514, 1941
12. Bernauer et al., Bionformatics, 5:555, 2007
13. Bernauer et al., Bioinformatics, 24:652, 2008
14. Bourquard et al., PLoS One, 6:e18541, 2011
15. Bourquard et al., Sci. Reports, 5 :10760, 2015
16. Fan et Hendrickson, Nature, 433:269, 2005

## Revendications

1. Ligand de l'hormone folliculo-stimulante (FSH) potentialisant la bioactivité de la FSH et de l'hormone gonadotropine chorionique (CG), **caractérisé en ce que** ledit ligand est un anticorps monoclonal ou un fragment de celui-ci et **en ce que** :
le domaine variable de la chaine lourde contient les CDRs suivants :
- VH-CDR1, défini par la séquence GFTFSDFG (SEQ ID NO : 5) ;
- VH-CDR2, défini par la séquence ISSGSSTR (SEQ ID NO : 6) ;
- VH-CDR3, défini par la séquence ARSYDGYHVPSFAY (SEQ ID NO : 7) ; et
le domaine variable de la chaine légère contient les CDRs suivants :
- VL-CDR1, défini par la séquence QNIVHSNGNTY (SEQ ID NO : 8) ;
- VL-CDR2, défini par la séquence RVS ;
- VL-CDR3, défini par la séquence FQGSHVPLT (SEQ ID NO : 9).

2. Ligand selon la revendication 1, **caractérisé en ce que** le ligand est choisi dans le groupe constitué de Fab, Fab', F(ab')2, Fv, dsFv, scFv, diabodies, triabodies, et tétrabodies.

3. Ligand selon la revendication 1, **caractérisé en ce que** le ligand est l'anticorps monoclonal A8 produit par l'hybridome CNCM I-4828.

4. Ligand selon la revendication 2, **caractérisé en ce que** la séquence peptidique du scFv est la séquence SEQ ID NO : 11.

5. Ligand selon l'une quelconque des revendications 1 à 4, pour une utilisation comme médicament.

6. Complexe ligand-gonadotrophine choisi parmi :
un complexe d'un ligand selon l'une quelconque des revendications 1 à 4 avec la FSH ou un peptide actif de celle-ci ;
un complexe d'un ligand selon l'une quelconque des revendications 1 à 4 avec l'hormone gonadotropine chorionique (CG) ou un peptide actif de celle-ci.

7. Complexe selon la revendication 6, pour une utilisation comme médicament.

8. Composition pharmaceutique comprenant un ligand selon l'une quelconque des revendications 1 à 4 et/ou un complexe selon la revendication 6 et un véhicule pharmaceutiquement acceptable.

9. Composition pharmaceutique selon la revendication 8, **caractérisée en ce qu'**elle comprend en outre une FSH et/ou une CG.

10. Composition pharmaceutique selon l'une quelconque des revendications 8 ou 9, pour une utilisation comme médicament.

11. Ligand selon l'une quelconque des revendications 1 à 4 ou complexe selon la revendication 6, pour une utilisation dans le traitement et/ou la prévention de l'infertilité ou de l'hypofertilité chez un mammifère.

## Patentansprüche

1. Ligand von follikelstimulierendem Hormon (FSH), der die Bioaktivität von FSH und Choriogonadotropin(CG)-Hormon verstärkt, **dadurch gekennzeichnet, dass** es sich bei dem Liganden um einen monoklonalen Antikörper oder ein Fragment davon handelt und dass:
die variable Domäne der schweren Kette die folgenden CDRs enthält:
- VH-CDR1, definiert durch die Sequenz GFTFSDFG (SEQ ID NO: 5);
- VH-CDR2, definiert durch die Sequenz ISSGSSTR (SEQ ID NO: 6);
- VH-CDR3, definiert durch die Sequenz ARSYDGYHVPSFAY (SEQ ID NO: 7); und
die variable Domäne der leichten Kette die folgenden CDRs enthält:
- VL-CDR1, definiert durch die Sequenz QNIVHSNGNTY (SEQ ID NO: 8);
- VL-CDR2, definiert durch die Sequenz RVS;
- VL-CDR3, definiert durch die Sequenz FQGSHVPLT (SEQ ID NO: 9).

2. Ligand nach Anspruch 1, **dadurch gekennzeichnet, dass** der Ligand aus der Gruppe bestehend aus Fab, Fab', F(Ab')2, Fv, dsFV, Diabodies, Triabodies und Tetrabodies ausgewählt ist.

3. Ligand nach Anspruch 1, **dadurch gekennzeichnet, dass** es sich bei dem Liganden um den durch das Hybridom CNCM 1-4828 produzierten monoklonalen A8-Antikörper handelt.

4. Ligand nach Anspruch 2, **dadurch gekennzeichnet, dass** es sich bei der Peptidsequenz von scFv um die Sequenz SEQ ID NO: 11 handelt.

5. Ligand nach einem der Ansprüche 1 bis 4 zur Verwendung als Medikament.

6. Ligand-Gonadotropin-Komplex, ausgewählt aus:
einem Komplex eines Liganden nach einem der Ansprüche 1 bis 4 mit FSH oder einem aktiven Peptid davon;
einem Komplex eines Liganden nach einem der Ansprüche 1 bis 4 mit Choriogonadotropin(CG)-Hormon oder einem aktiven Peptid davon.

7. Komplex nach Anspruch 6 zur Verwendung als Medikament.

8. Pharmazeutische Zusammensetzung, umfassend einen Liganden nach einem der Ansprüche 1 bis 4 und/oder einen Komplex nach Anspruch 6 und ein pharmazeutisch unbedenkliches Vehikel.

9. Pharmazeutische Zusammensetzung nach Anspruch 8, **dadurch gekennzeichnet, dass** sie außerdem ein FSH und/oder ein CG umfasst.

10. Pharmazeutische Zusammensetzung nach Anspruch 8 oder 9 zur Verwendung als Medikament.

11. Ligand nach einem der Ansprüche 1 bis 4 oder Komplex nach Anspruch 6 zur Verwendung bei der Behandlung und/oder Prävention von Unfruchtbarkeit oder verminderter Fruchtbarkeit bei einem Säugetier.

## Claims

1. Follicle-stimulating hormone (FSH) ligand which potentiates the bioactivity of FSH and of chorionic gonadotropin (CG), **characterized in that** said ligand is a monoclonal antibody or a fragment thereof and **in that**:
the heavy chain variable domain contains the following CDRs:
- VH-CDR1, defined by the sequence GFTFSDFG (SEQ ID NO: 5);
- VH-CDR2, defined by the sequence ISSGSSTR (SEQ ID NO: 6);
- VH-CDR3, defined by the sequence ARSYDGYHVPSFAY (SEQ ID NO: 7); and
the light chain variable domain contains the following CDRs:
- VL-CDR1, defined by the sequence QNIVHSNGNTY (SEQ ID NO: 8);
- VL-CDR2, defined by the sequence RVS;
- VL-CDR3, defined by the sequence FQGSHVPLT (SEQ ID NO: 9).

2. Ligand as claimed in claim 1, **characterized in that** the ligand is chosen from the group consisting of: Fab, Fab', F(ab')2, Fv, dsFv, scFv, diabodies, triabodies, and tetrabodies.

3. Ligand as claimed in claim 1, **characterized in that** the ligand is the A8 monoclonal antibody produced by the CNCM I-4828 hybridoma.

4. Ligand as claimed in claim 2, **characterized in that** the peptide sequence of the scFv is the sequence SEQ ID NO: 11.

5. Ligand as claimed in any one of claims 1 to 4, for use as a medicament.

6. Ligand-gonadotropin complex chosen from:
a complex of a ligand as claimed in any one of claims 1 to 4 with FSH or an active peptide thereof;
a complex of a ligand as claimed in any one of claims 1 to 4 with the chorionic gonadotropin (CG) hormone, or an active peptide thereof.

7. Complex as claimed in claim 6, for use as a medicament.

8. Pharmaceutical composition comprising a ligand as claimed in any one of claims 1 to 4 and/or a complex as claimed in claim 6 and a pharmaceutically acceptable carrier.

9. Pharmaceutical composition as claimed in claim 8, **characterized in that** it also comprises a FSH and/or a CG.

10. Pharmaceutical composition as claimed in either one of claims 8 or 9, for use as a medicament.

11. Ligand as claimed in any one of claims 1 to 4 or complex as claimed in claim 6, for use in the treatment and/or prevention of infertility or of hypofertility in a mammal.


### SEQUENCE LISTING

<110> REPROPHARM
   INRA
   MAUREL, Marie-Christine
   POUPON, Anne
   REITER, Eric
   GEGOT, Gwenhael
   DURAND, Guillaume
   KARA, Elodie
   CASTERET, Sophie
<120> LIGAND POTENTIALISANT DE LA BIOACTIVITE DE LA FSH
<130> BNT217763PC00
<150> FR1458463
   <151> 2014-09-10
<160> 38
<170> Patent In version 3.5
<210> 1
   <211> 364
   <212> DNA
   <213> Artificial Sequence
<220>
   <223> VH A8
<400> 1
   gaggtccaac tgcaggagtc agggggaggc ttagtgcagc ctggagggtc ccggaaactc       60
   tcctgtgcag cctctggatt cactttcagt gactttggaa cgcactggat tcgtcaggct      120
   ccagagaagg gactggagtg ggtcgcatat attagtagtg gcagtagtac caggtactat      180
   gcagacacag tgaagggccg attcaccatc tccagagacg atcccaagaa caccctgttc      240
   ctgcaaatga ccagtctaag gtctgaagac acggccatgt attactgtgc aagatcttat      300
   gatggttacc acgttccctc ctttgcttac tggggccaag ggactctggt cactgtctct      360
   gcag                                                                   364
<210> 2
   <211> 121
   <212> PRT
   <213> Artificial Sequence
<220>
   <223> VH A8
<400> 2
   Glu Val Gln Leu Gln Glu Ser Gly Gly Gly Leu Val Gln Pro Gly Gly
   1               5                   10                  15
   Ser Arg Lys Leu Ser Cys Ala Ala Ser Gly Phe Thr Phe Ser Asp Phe
               20                  25                  30
   Gly Thr His Trp Ile Arg Gln Ala Pro Glu Lys Gly Leu Glu Trp Val
           35                  40                  45
   Ala Tyr Ile Ser Ser Gly Ser Ser Thr Arg Tyr Tyr Ala Asp Thr Val
       50                  55                  60
   Lys Gly Arg Phe Thr Ile Ser Arg Asp Asp Pro Lys Asn Thr Leu Phe
   65                  70                  75                  80
   Leu Gln Met Thr Ser Leu Arg Ser Glu Asp Thr Ala Met Tyr Tyr Cys
                   85                  90                  95
   Ala Arg Ser Tyr Asp Gly Tyr His Val Pro Ser Phe Ala Tyr Trp Gly
               100                 105                 110
   Gln Gly Thr Leu Val Thr Val Ser Ala
           115                 120
<210> 3
   <211> 336
   <212> DNA
   <213> Artificial Sequence
<220>
   <223> VL A8
<400> 3
   gatgttttga tgacccaaac tccactctcc ctgtctgtca gtcttggaga tcaagcctcc       60
   atctcttgca gatctagtca gaacattgta catagtaatg gaaacaccta tttagaatgg      120
   tacctgcaga aaccaggcca gtctccaaag ctcctgatct acagagtttc caaccgattt      180
   tctgggatcc cagacaggtt cagtggcagt tcatcaggga cagacttcac actcaagatc      240
   agcagagtgg aggctgagga tctgggagtt tattactgct ttcaaggttc acatgttccg      300
   ctcacattcg gtgctgggac caagctggag ctgaaa                                336
<210> 4
   <211> 112
   <212> PRT
   <213> Artificial Sequence
<220>
   <223> VL A8
<400> 4
   Asp Val Leu Met Thr Gln Thr Pro Leu Ser Leu Ser Val Ser Leu Gly
   1               5                   10                  15
   Asp Gln Ala Ser Ile Ser Cys Arg Ser Ser Gln Asn Ile Val His Ser
               20                  25                  30
   Asn Gly Asn Thr Tyr Leu Glu Trp Tyr Leu Gln Lys Pro Gly Gln Ser
           35                  40                  45
   Pro Lys Leu Leu Ile Tyr Arg Val Ser Asn Arg Phe Ser Gly Ile Pro
       50                  55                  60
   Asp Arg Phe Ser Gly Ser Ser Ser Gly Thr Asp Phe Thr Leu Lys Ile
   65                  70                  75                  80
   Ser Arg Val Glu Ala Glu Asp Leu Gly Val Tyr Tyr Cys Phe Gln Gly
                   85                  90                  95
   Ser His Val Pro Leu Thr Phe Gly Ala Gly Thr Lys Leu Glu Leu Lys
               100                 105                 110
<210> 5
   <211> 8
   <212> PRT
   <213> Artificial Sequence
<220>
   <223> VH-CDR1
<400> 5
   Gly Phe Thr Phe Ser Asp Phe Gly
   1               5
<210> 6
   <211> 8
   <212> PRT
   <213> Artificial Sequence
<220>
   <223> VH-CDR2
<400> 6
   Ile Ser Ser Gly Ser Ser Thr Arg
   1               5
<210> 7
   <211> 14
   <212> PRT
   <213> Artificial Sequence
<220>
   <223> VH-CDR3
<400> 7
   Ala Arg Ser Tyr Asp Gly Tyr His Val Pro Ser Phe Ala Tyr
   1               5                   10
<210> 8
   <211> 11
   <212> PRT
   <213> Artificial Sequence
<220>
   <223> VL-CDR1
<400> 8
   Gln Asn Ile Val His Ser Asn Gly Asn Thr Tyr
   1               5                   10
<210> 9
   <211> 9
   <212> PRT
   <213> Artificial Sequence
<220>
   <223> VL-CDR3
<400> 9
   Phe Gln Gly Ser His Val Pro Leu Thr
   1               5
<210> 10
   <211> 780
   <212> DNA
   <213> Artificial Sequence
<220>
   <223> scFv A8
<400> 10
   caggtgcagc tgcagcagag cggtggcggt ctggtacaac cgggtggcag ccgtaaactg      60
   agctgcgcgg catcaggctt taccttttca gactttggca ctcattggat tcgtcaggcc     120
   ccggaaaagg gcctggaatg ggtagcgtac attagcagcg gctcaagcac tcgctattat     180
   gccgatacgg tcaaaggccg ctttacgatt tcgcgtgatg atccgaaaaa caccctgttc     240
   ctgcaaatga cgtcgctgcg tagcgaagat actgccatgt attactgtgc gcgttcatat     300
   gatggttacc atgtcccgtc gttcgcgtat tggggccagg gcaccctggt gactgtgagc     360
   gcaggtggtg gcggttcagg tggtggtggc tcaggtggcg gtggtagcga tattcagatg     420
   acccagaccc cgctgtcgct gagcgtttca ctgggtgacc aggcaagcat ctcatgtcgc     480
   agcagccaga acatcgtgca ctcgaatggt aatacctacc tggaatggta cctgcaaaag     540
   ccgggccaaa gccctaaact gctgatctat cgtgtgtcga accgcttttc gggcattcct     600
   gatcgcttca gcggcagctc gtcaggcacc gacttcaccc tgaaaatttc gcgcgttgag     660
   gccgaagacc tgggtgtata ttactgcttc cagggttcgc acgttccgct gacgtttggc     720
   gcgggcacga aactcgagct gaaacaccat caccatcacc attaactcga gctgaagtaa     780
<210> 11
   <211> 254
   <212> PRT
   <213> Artificial Sequence
<220>
   <223> scFv A8
<400> 11
   Gln Val Gln Leu Gln Gln Ser Gly Gly Gly Leu Val Gln Pro Gly Gly
   1               5                   10                  15
   Ser Arg Lys Leu Ser Cys Ala Ala Ser Gly Phe Thr Phe Ser Asp Phe
               20                  25                  30
   Gly Thr His Trp Ile Arg Gln Ala Pro Glu Lys Gly Leu Glu Trp Val
           35                  40                  45
   Ala Tyr Ile Ser Ser Gly Ser Ser Thr Arg Tyr Tyr Ala Asp Thr Val
       50                  55                  60
   Lys Gly Arg Phe Thr Ile Ser Arg Asp Asp Pro Lys Asn Thr Leu Phe
   65                  70                  75                  80
   Leu Gln Met Thr Ser Leu Arg Ser Glu Asp Thr Ala Met Tyr Tyr Cys
                   85                  90                  95
   Ala Arg Ser Tyr Asp Gly Tyr His Val Pro Ser Phe Ala Tyr Trp Gly
               100                 105                 110
   Gln Gly Thr Leu Val Thr Val Ser Ala Gly Gly Gly Gly Ser Gly Gly
           115                 120                 125
   Gly Gly Ser Gly Gly Gly Gly Ser Asp Ile Gln Met Thr Gln Thr Pro
       130                 135                 140
   Leu Ser Leu Ser Val Ser Leu Gly Asp Gln Ala Ser Ile Ser Cys Arg
   145                 150                 155                 160
   Ser Ser Gln Asn Ile Val His Ser Asn Gly Asn Thr Tyr Leu Glu Trp
                   165                 170                 175
   Tyr Leu Gln Lys Pro Gly Gln Ser Pro Lys Leu Leu Ile Tyr Arg Val
               180                 185                 190
   Ser Asn Arg Phe Ser Gly Ile Pro Asp Arg Phe Ser Gly Ser Ser Ser
           195                 200                 205
   Gly Thr Asp Phe Thr Leu Lys Ile Ser Arg Val Glu Ala Glu Asp Leu
       210                 215                 220
   Gly Val Tyr Tyr Cys Phe Gln Gly Ser His Val Pro Leu Thr Phe Gly
   225                 230                 235                 240
   Ala Gly Thr Lys Leu Glu Leu Lys His His His His His His
                   245                 250
<210> 12
   <211> 35
   <212> DNA
   <213> Artificial Sequence
<220>
   <223> vHRev1
<400> 12
   cgggatcctc tagaggtcca actgcaggag tcagg 35
<210> 13
   <211> 37
   <212> DNA
   <213> Artificial Sequence
<220>
   <223> VHRev2
<400> 13
   agatctagaa agcttaggtc aagctgcagc agtcagg 37
<210> 14
   <211> 19
   <212> DNA
   <213> Artificial Sequence
<220>
   <223> VHFor1
<400> 14
   caggggccag tggatagac 19
<210> 15
   <211> 32
   <212> DNA
   <213> Artificial Sequence
<220>
   <223> VHFor2
<400> 15
   cgggatcctc tagacagtgg ataracmgat gg 32
<210> 16
   <211> 49
   <212> DNA
   <213> Artificial Sequence
<220>
   <223> VHFor3
<400> 16
   agatctagag aattctgagg agacggtgac cgtggtccct tggccccag 49
<210> 17
   <211> 21
   <212> DNA
   <213> Artificial Sequence
<220>
   <223> MKRev1
<400> 17
   gatgttttga tgacccaaac t 21
<210> 18
   <211> 21
   <212> DNA
   <213> Artificial Sequence
<220>
   <223> MKRev7
<400> 18
   gatatccaga tgacacagac t 21
<210> 19
   <211> 21
   <212> DNA
   <213> Artificial Sequence
<220>
   <223> MKC5For
<400> 19
   ggatacagtt ggtgcagcat c 21
<210> 20
   <211> 30
   <212> DNA
   <213> Artificial Sequence
<220>
   <223> A8_VL4
<400> 20
   atgaagttgc ctgttaggct gttggtgctg 30
<210> 21
   <211> 31
   <212> DNA
   <213> Artificial Sequence
<220>
   <223> A8-VL5
<400> 21
   gctgatgttc tggattcctg cttccagcag t 31
<210> 22
   <211> 30
   <212> DNA
   <213> Artificial Sequence
<220>
   <223> A8-VL6
<400> 22
   atgatgagtc ctgcccagtt cctgtttctg 30
<210> 23
   <211> 27
   <212> DNA
   <213> Artificial Sequence
<220>
   <223> A8-VLrev
<400> 23
   tgtgagcgga acatgtgaac cttgaaa 27
<210> 24
   <211> 15
   <212> PRT
   <213> Artificial Sequence
<220>
   <223> Lieur
<400> 24
   Gly Gly Gly Gly Ser Gly Gly Gly Gly Ser Gly Gly Gly Gly Ser
   1               5                   10                  15
<210> 25
   <211> 16
   <212> PRT
   <213> Artificial Sequence
<220>
   <223> Fragment VL du scFv
<400> 25
   Leu Glu Ile Lys His His His His His His Leu Glu Ile Lys Val Asp
   1               5                   10                  15
<210> 26
   <211> 92
   <212> PRT
   <213> Homo sapiens
<220>
   <221> misc_feature
   <223> sous-unité alpha hFSH, hCG et hLH
<220>
   <221> misc_feature
   <222> (17)..(17)
   <223> Xaa = Leu ou Phe
<400> 26
   Ala Pro Asp Val Gln Asp Cys Pro Glu Cys Thr Leu Gln Glu Asn Pro
   1               5                   10                  15
   Xaa Phe Ser Gln Pro Gly Ala Pro Ile Leu Gln Cys Met Gly Cys Cys
               20                  25                  30
   Phe Ser Arg Ala Tyr Pro Thr Pro Leu Arg Ser Lys Lys Thr Met Leu
           35                  40                  45
   Val Gln Lys Asn Val Thr Ser Glu Ser Thr Cys Cys Val Ala Lys Ser
       50                  55                  60
   Tyr Asn Arg Val Thr Val Met Gly Gly Phe Lys Val Glu Asn His Thr
   65                  70                  75                  80
   Ala Cys His Cys Ser Thr Cys Tyr Tyr His Lys Ser
                   85                  90
<210> 27
   <211> 96
   <212> PRT
   <213> Ovis aries
<220>
   <221> misc_feature
   <223> Sous-unité alpha oLHet oFSH
<400> 27
   Phe Pro Asp Gly Glu Phe Thr Met Gln Gly Cys Pro Glu Cys Lys Leu
   1               5                   10                  15
   Lys Glu Asn Lys Tyr Phe Ser Lys Pro Asp Ala Pro Ile Tyr Gln Cys
               20                  25                  30
   Met Gly Cys Cys Phe Ser Arg Ala Tyr Pro Thr Pro Ala Arg Ser Lys
           35                  40                  45
   Lys Thr Met Leu Val Pro Lys Asn Ile Thr Ser Glu Ala Thr Cys Cys
       50                  55                  60
   Val Ala Lys Ala Phe Thr Lys Ala Thr Val Met Gly Asn Val Arg Val
   65                  70                  75                  80
   Glu Asn His Thr Glu Cys His Cys Ser Thr Cys Tyr Tyr His Lys Ser
                   85                  90                  95
<210> 28
   <211> 96
   <212> PRT
   <213> Sus scrofa
<220>
   <221> misc_feature
   <223> sous-unité alpha pLH et pFSH
<400> 28
   Phe Pro Asp Gly Glu Phe Thr Met Gln Gly Cys Pro Glu Cys Lys Leu
   1               5                   10                  15
   Lys Glu Asn Lys Tyr Phe Ser Lys Leu Gly Ala Pro Ile Tyr Gln Cys
               20                  25                  30
   Met Gly Cys Cys Phe Ser Arg Ala Tyr Pro Thr Pro Ala Arg Ser Lys
           35                  40                  45
   Lys Thr Met Leu Val Pro Lys Asn Ile Thr Ser Glu Ala Thr Cys Cys
       50                  55                  60
   Val Ala Lys Ala Phe Thr Lys Ala Thr Val Met Gly Asn Ala Arg Val
   65                  70                  75                  80
   Glu Asn His Thr Glu Cys His Cys Ser Thr Cys Tyr Tyr His Lys Ser
                   85                  90                  95
<210> 29
   <211> 111
   <212> PRT
   <213> Homo sapiens
<220>
   <221> misc_feature
   <223> sous-unité bêta hFSH
<400> 29
   Asn Ser Cys Glu Leu Thr Asn Ile Thr Ile Ala Ile Glu Lys Glu Glu
   1               5                   10                  15
   Cys Arg Phe Cys Ile Ser Ile Asn Thr Thr Trp Cys Ala Gly Tyr Cys
               20                  25                  30
   Tyr Thr Arg Asp Leu Val Tyr Lys Asp Pro Ala Arg Pro Lys Ile Gln
           35                  40                  45
   Lys Thr Cys Thr Phe Lys Glu Leu Val Tyr Glu Thr Val Arg Val Pro
       50                  55                  60
   Gly Cys Ala His His Ala Asp Ser Leu Tyr Thr Tyr Pro Val Ala Thr
   65                  70                  75                  80
   Gln Cys His Cys Gly Lys Cys Asp Ser Asp Ser Thr Asp Cys Thr Val
                   85                  90                  95
   Arg Gly Leu Gly Pro Ser Tyr Cys Ser Phe Gly Glu Met Lys Glu
               100                 105                 110
<210> 30
   <211> 132
   <212> PRT
   <213> Homo sapiens
<220>
   <221> misc_feature
   <223> sous-unité bêta hCG
<400> 30
   Ser Lys Glu Pro Leu Arg Pro Arg Cys Arg Pro Ile Asn Ala Thr Leu
   1               5                   10                  15
   Ala Val Glu Lys Glu Gly Cys Pro Val Cys Ile Thr Val Asn Thr Thr
               20                  25                  30
   Ile Cys Ala Gly Tyr Cys Pro Thr Met Thr Arg Val Leu Gln Gly Val
           35                  40                  45
   Leu Pro Ala Leu Pro Gln Val Val Cys Asn Tyr Arg Asp Val Arg Phe
       50                  55                  60
   Glu Ser Ile Arg Leu Pro Gly Cys Pro Arg Gly Val Asn Pro Val Val
   65                  70                  75                  80
   Ser Tyr Ala Val Ala Leu Ser Cys Gln Cys Ala Leu Cys Arg Arg Ser
                   85                  90                  95
   Thr Thr Asp Cys Gly Gly Pro Lys Asp His Pro Leu Thr Cys Asp Asp
               100                 105                 110
   Pro Arg Phe Gln Asp Ser Ser Ser Ser Lys Ala Pro Pro Pro Ser Leu
           115                 120                 125
   Pro Ser Pro Ser
       130
<210> 31
   <211> 123
   <212> PRT
   <213> Homo sapiens
<220>
   <221> misc_feature
   <223> sous-unité bêta hLH
<400> 31
   Ser Arg Glu Pro Leu Arg Pro Arg Pro Trp Cys His Pro Ile Asn Ala
   1               5                   10                  15
   Ile Leu Ala Val Glu Lys Glu Gly Cys Pro Val Cys Ile Thr Val Asn
               20                  25                  30
   Thr Thr Ile Cys Ala Gly Tyr Cys Pro Thr Met Met Arg Val Leu Gln
           35                  40                  45
   Ala Val Leu Pro Pro Leu Pro Gln Val Val Cys Thr Tyr Arg Asp Val
       50                  55                  60
   Arg Phe Glu Ser Ile Arg Leu Pro Gly Cys Pro Arg Gly Val Asp Pro
   65                  70                  75                  80
   Val Val Ser Phe Pro Val Ala Leu Ser Cys Arg Cys Gly Pro Cys Arg
                   85                  90                  95
   Arg Ser Thr Ser Asp Cys Gly Gly Pro Lys Asp His Pro Leu Thr Cys
               100                 105                 110
   Asp His Pro Gln Leu Ser Gly Leu Leu Phe Leu
           115                 120
<210> 32
   <211> 121
   <212> PRT
   <213> Ovis aries
<220>
   <221> misc_feature
   <223> sous-unité bêta oLH
<400> 32
   Ser Arg Gly Pro Leu Arg Pro Leu Cys Gln Pro Ile Asn Ala Thr Leu
   1               5                   10                  15
   Ala Ala Glu Lys Glu Ala Cys Pro Val Cys Ile Thr Phe Thr Thr Ser
               20                  25                  30
   Ile Cys Ala Gly Tyr Cys Leu Ser Met Lys Arg Val Leu Pro Val Ile
           35                  40                  45
   Leu Pro Pro Met Pro Gln Arg Val Cys Thr Tyr His Glu Leu Arg Phe
       50                  55                  60
   Ala Ser Val Arg Leu Pro Gly Cys Pro Pro Gly Val Asp Pro Met Val
   65                  70                  75                  80
   Ser Phe Pro Val Ala Leu Ser Cys His Cys Gly Pro Cys Arg Leu Ser
                   85                  90                  95
   Ser Thr Asp Cys Gly Gly Pro Arg Thr Gln Pro Leu Ala Cys Asp His
               100                 105                 110
   Pro Pro Leu Pro Asp Ile Leu Phe Leu
           115                 120
<210> 33
   <211> 121
   <212> PRT
   <213> Sus scrofa
<220>
   <221> misc_feature
   <223> sous-unité bêta pLH
<400> 33
   Ser Arg Gly Pro Leu Arg Pro Leu Cys Arg Pro Ile Asn Ala Thr Leu
   1               5                   10                  15
   Ala Ala Glu Asn Glu Ala Cys Pro Val Cys Ile Thr Phe Thr Thr Ser
               20                  25                  30
   Ile Cys Ala Gly Tyr Cys Pro Ser Met Val Arg Val Leu Pro Ala Ala
           35                  40                  45
   Leu Pro Pro Val Pro Gln Pro Val Cys Thr Tyr Arg Glu Leu Ser Phe
       50                  55                  60
   Ala Ser Ile Arg Leu Pro Gly Cys Pro Pro Gly Val Asp Pro Thr Val
   65                  70                  75                  80
   Ser Phe Pro Val Ala Leu Ser Cys His Cys Gly Pro Cys Arg Leu Ser
                   85                  90                  95
   Ser Ser Asp Cys Gly Gly Pro Arg Ala Gln Pro Leu Ala Cys Asp Arg
               100                 105                 110
   Pro Leu Leu Pro Gly Leu Leu Phe Leu
           115                 120
<210> 34
   <211> 110
   <212> PRT
   <213> Ovis aries
<220>
   <221> misc_feature
   <223> sous-unité bêta oFSH
<400> 34
   Ser Cys Glu Leu Thr Asn Ile Thr Ile Thr Val Glu Lys Glu Glu Cys
   1               5                   10                  15
   Ser Phe Cys Ile Ser Ile Asn Thr Thr Trp Cys Ala Gly Tyr Cys Tyr
               20                  25                  30
   Thr Arg Asp Leu Val Tyr Lys Asp Pro Ala Arg Pro Asn Ile Gln Lys
           35                  40                  45
   Ala Cys Thr Phe Lys Glu Leu Val Tyr Glu Thr Val Lys Val Pro Gly
       50                  55                  60
   Cys Ala His His Ala Asp Ser Leu Tyr Thr Tyr Pro Val Ala Thr Glu
   65                  70                  75                  80
   Cys His Cys Gly Lys Cys Asp Arg Asp Ser Thr Asp Cys Thr Val Arg
                   85                  90                  95
   Gly Leu Gly Pro Ser Tyr Cys Ser Phe Ser Asp Ile Arg Glu
               100                 105                 110
<210> 35
   <211> 109
   <212> PRT
   <213> Sus scrofa
<220>
   <221> misc_feature
   <223> sous-unité bêta pFSH
<400> 35
   Cys Glu Leu Thr Asn Ile Thr Ile Thr Val Glu Lys Glu Glu Cys Gly
   1               5                   10                  15
   Phe Cys Ile Ser Ile Asn Thr Thr Trp Cys Ala Gly Tyr Cys Tyr Thr
               20                  25                  30
   Arg Asp Leu Val Tyr Lys Asp Pro Ala Arg Pro Asn Ile Gln Lys Thr
           35                  40                  45
   Cys Thr Phe Lys Glu Leu Val Tyr Glu Thr Val Lys Val Pro Gly Cys
       50                  55                  60
   Ala His His Ala Asp Ser Leu Tyr Thr Tyr Pro Val Ala Thr Glu Cys
   65                  70                  75                  80
   His Cys Gly Lys Cys Asp Ser Asp Ser Thr Asp Cys Thr Val Arg Gly
                   85                  90                  95
   Leu Gly Pro Ser Tyr Cys Ser Phe Ser Glu Met Lys Glu
               100                 105
<210> 36
   <211> 49
   <212> PRT
   <213> Homo sapiens
<220>
   <221> misc_feature
   <223> région N terminale du récepteur de la hFSH
<400> 36
   Cys His His Arg Ile Cys His Cys Ser Asn Arg Val Phe Leu Cys Gln
   1               5                   10                  15
   Glu Ser Lys Val Thr Glu Ile Pro Ser Asp Leu Pro Arg Asn Ala Ile
               20                  25                  30
   Glu Leu Arg Phe Val Leu Thr Lys Leu Arg Val Ile Gln Lys Gly Ala
           35                  40                  45
   Phe
<210> 37
   <211> 6
   <212> PRT
   <213> Artificial Sequence
<220>
   <223> région épitopique de A8 sur alphaFSH
<400> 37
   Gln Asp Cys Pro Glu Thr
   1 5
<210> 38
   <211> 4
   <212> PRT
   <213> Artificial Sequence
<220>
   <223> région épitopique de A8 sur bêtaFSH
<400> 38
   Ser Cys Glu Thr
   1